# FASCICULE DE BREVET EUROPEEN

(11) **EP 3 320 342 B1**
(45) Date de publication et mention de la délivrance du brevet: **03.03.2021**
(21) Numéro de dépôt: 16736458.7
(22) Date de dépôt: 07.07.2016
(51) Int. Cl.: G01N 33/543, A61L 27/52, C07F 7/18

(54) **NOUVEAUX HYDROGELS DE STRUCTURE SILYLÉE ET PROCÉDÉ D'OBTENTION**
NEUE HYDROGELE MIT SILYLIERTE STRUKTUR UND VERFAHREN ZUR HERSTELLUNG DAVON
NEW HYDROGELS HAVING A SILYLATED STRUCTURE, AND METHOD FOR OBTAINING SAME

(30) Priorité: 10.07.2015 FR 1556628
(43) Date de publication de la demande: 16.05.2018
(73) Titulaire: Université de Montpellier, 34090 Montpellier (FR); Centre National de la Recherche Scientifique (C.N.R.S.), 75016 Paris (FR)
(72) Inventeur: MARTINEZ, Jean, 34720 Caux (FR); MEHDI, Ahmad, 34090 Montpellier (FR); SUBRA, Gilles, 34980 Saint Gely Du Fesc (FR); JEBORS, Said, 34830 Jacou (FR); ECHALIER, Cécile, 34090 Montpellier (FR)
(74) Mandataire: Regimbeau
(86) Numéro de dépôt international: PCT/EP2016/066215
(87) Numéro de publication internationale: WO 2017/009200

(56) Documents cités:
- WO-A1-2011/089267
- WO-A1-2013/190148
- SEONGBONG JO ET AL: "novel poly(ethylene glycol) hydrogels from silylated PEGs", JOURNAL OF BIOACTIVE AND COMPATIBLE POLYMERS, SAGE, US, vol. 14, 1 novembre 1999 (1999-11-01), pages 457-473, XP008134058, ISSN: 0883-9115
- AIRONG SONG ET AL: "Antibacterial and cell-adhesive polypeptide and poly(ethylene glycol) hydrogel as a potential scaffold for wound healing", ACTA BIOMATERIALIA, ELSEVIER, AMSTERDAM, NL, vol. 8, no. 1, 5 octobre 2011 (2011-10-05) , pages 41-50, XP028125820, ISSN: 1742-7061, DOI: 10.1016/J.ACTBIO.2011.10.004 [extrait le 2011-10-11]
- BABAK RADI ET AL: "Controlled Poly(ethylene glycol) Network Structures through Silsesquioxane Cross-Links Formed by Sol-Gel Reactions", MACROMOLECULES, vol. 43, no. 23, 14 décembre 2010 (2010-12-14), pages 9957-9963, XP055243505, US ISSN: 0024-9297, DOI: 10.1021/ma101511p
- BABAK RADI ET AL: "Effect of dangling chains on the structure and physical properties of a tightly crosslinked poly(ethylene glycol) network", SOFT MATTER, vol. 9, no. 12, 1 janvier 2013 (2013-01-01), page 3262, XP055243509, GB ISSN: 1744-683X, DOI: 10.1039/c3sm27819k
- LI JING ET AL: "Synthesis of polyethylene glycol (PEG) derivatives and PEGylated-peptide biopolymer conjugates", BIOMACROMOLECULES, AMERICAN CHEMICAL SOCIETY, US, vol. 4, no. 4, 17 mai 2003 (2003-05-17), pages 1055-1067, XP002328259, ISSN: 1525-7797, DOI: 10.1021/BM034069L

## Description

L'objet de la présente invention concerne des hydrogels préparés au moyen de molécules organiques silylées (telles que des biomolécules silylées), leur procédé d'obtention et leurs utilisations.

### Introduction

Il existe une recherche constante afin de trouver de nouveaux hydrogels, pouvant être fonctionnalisés, présentant des propriétés physico-chimiques ou biologiques précises, en particulier pour la médecine humaine.

Toutefois, il existe actuellement de nombreuses contraintes techniques avec ces matériaux. Par exemple, pour obtenir des matériaux possédant des propriétés biologiques, des biomolécules (peptides, protéines, saccharides, oligonucléotides, etc...) sont souvent introduites dans ces matériaux de façon non covalente. Cette façon de faire est certes une technique d'introduction certaine des molécules actives dans les hydrogels présentant ainsi *ab initio* les propriétés biologiques et rhéologiques voulues. Elle présente néanmoins le désavantage que les hydrogels ainsi obtenus ne peuvent être envisagés pour être utilisés *in vivo,* par exemple placés dans un patient, sans une libération de la/des molécules actives incorporées au sein de leur matrice. En outre, les hydrogels étant déjà formés et très visqueux (dans le meilleur des cas), leur administration par injection est très douloureuse pour le patient.

Ainsi, si l'on désire que l'hydrogel conserve une activité biologique sans relargage intempestif, la liaison entre l'actif et la matrice de l'hydrogel doit être covalente. Toutefois, lier de manière covalente des biomolécules dans ces hydrogels sans en altérer fondamentalement les propriétés physico-chimiques reste difficile pour plusieurs raisons : les biomolécules étant généralement des molécules dont la chimie (de synthèse) est souvent particulière (e.g. des molécules possédant souvent de nombreuses fonctions chimiques réactives), il est difficile de ne pas impliquer des réactions secondaires lors de la formation de l'hydrogel fonctionnalisé. En outre, il est difficile de lier de manière covalente ces biomolécules (souvent volumineuses) jusqu'au cœur de la matrice sans en altérer les propriétés physico-chimiques et/ou son intégrité quand on réalise une fonctionnalisation directe de l'hydrogel.

Le document brevet WO2011089267 traite, parmi d'autres sujets, de la fabrication d'hydrogels auxquels sont liées de manière covalente des biomolécules actives. Pour ce faire, c'est la chimie du silicium qui a été choisie complémentairement aux biomolécules choisies. Ainsi, deux types de biomolécules silylées sont synthétisées, l'une ayant pour rôle la matrice structurelle de l'hydrogel (en particulier HPMC silylé dans WO2011089267), et l'autre ayant le rôle de fonctionnalisation biologique active de l'hydrogel. L'invention décrite dans WO2011089267 apparaît donc séduisante. Toutefois, l'invention divulguée ne résout pas tous les problèmes techniques évoqués ci-dessus.

D'une part les propriétés rhéologiques des hydrogels sont difficilement contrôlables par la technique de WO2011089267. En effet, WO2011089267 divulgue des hydrogels formés à partir d'une « biomolécule » possédant un seul groupement silylé, dont les propriétés vont essentiellement dépendre de la nature du fragment « biomolécule ». Ceci est un handicap car il est important de pouvoir finement contrôler la nature chimique de la matrice de l'hydrogel indépendamment de ses propriétés rhéologiques. Il est en outre fondamental que l'hydrogel assure par exemple une biocompatibilité selon le modèle étudié et/ou présente des fonctions biologiques et/ou physico-chimiques particulières voulues selon le domaine concerné. L'un des aspects selon la présente invention a été donc de faciliter le contrôle de la rhéologie des hydrogels.

De plus, seul un procédé de fabrication des intermédiaires de synthèse (biomolécules silylées) est donné dans WO2011089267 (exemple 1 dudit document) et implique la suspension dans un solvant comme de l'acétonitrile anhydre de la biomolécule d'intérêt à silyler. Ceci ne permet donc pas d'obtenir toutes les biomolécules silylées voulues. La purification apparaît donc être un réel problème quasiment non abordé dans WO2011089267. En effet, la purification décrite implique des lavages successifs du solide suspendu dans de l'acétonitrile afin d'en retirer toutes les impuretés. Or, une telle purification ne permet pas l'obtention d'un produit (principe actif) d'une pureté nécessaire pour une utilisation médicale, et d'autre part nécessite que le produit obtenu soit également insoluble dans le solvant de lavage (acétonitrile anhydre dans WO2011089267). Les techniques de purification usuelles telle que la chromatographie en phase inverse (technique permettant une purification optimale pour la majorité des biomolécules) semblent être exclues de la technologie de WO2011089267 car les molécules silylées décrites sont hautement réactives en milieu aqueux, résultant ainsi en une polymérisation une fois introduites dans une colonne HPLC en présence d'eau.

Ainsi, l'enseignement de WO2011089267, séduisant en première approche, ne permet pas de finement travailler avec les constituants des hydrogels afin de proposer des hydrogels fonctionnalisés de qualité médicale ou d'une qualité suffisante pour des domaines voisins (recherche biomédicale en particulier).

Toutefois, même s'il existe d'autres exemples de molécules organiques silylées dans la littérature, tels que ceux trouvés dans WO2013190148 où est divulgué des conjugués peptidiques silylés, il n'est en revanche pas divulgué leur utilisation pour la fabrication d'hydrogels, ou une méthode de purification optimale impliquant par exemple l'HPLC en phase inverse. En outre, seuls des « xérogels », formes non hydratées de gels, sont divulgués à cette occasion. Or, le contrôle de la rhéologie d'hydrogels est également dépendant de leur taux d'hydratation. Les documents Seongbong et al., Journal of Bioactive and Compatible Polymers, vol. 14, Nov. 1999, pages 457-473, Babak et al., Macromolecules, vol. 43, no 23, 2010, pages 9957-9963 et Babak et al., Soft Matter, vol. 9, no 12, 2013, page 3262-3271 décrivent tous des procédés de fabrication d'hydrogels bi-silylés en milieu acide.

En résumé, les hydrogels ont un potentiel d'utilisation considérable, en particulier dans le domaine médical. Néanmoins ce potentiel est actuellement limité par un mauvais contrôle de la fabrication de ces hydrogels, une méconnaissance des moyens de contrôle des propriétés rhéologiques de ces hydrogels ou simplement l'impossibilité de réaliser des hydrogels biocompatibles actifs dont l'activité perdure dans le temps.

Ainsi, l'objet de la présente demande de brevet est de proposer au moins une solution technique pour pallier les méthodes incomplètes de l'art antérieur afin d'aboutir à l'obtention d'hydrogels facilement modulables, pouvant en particulier être utilisés dans le domaine médical et les domaines voisins.

Afin de surmonter les problèmes rhéologiques des hydrogels, tout en permettant d'intégrer les (bio-)molécules voulues dans leur structure, il a été découvert de manière surprenante qu'en travaillant avec des (bio-)molécules au moins bi-silylées, il était possible de modifier les propriétés rhéologiques des gels quasiment indépendamment de la nature des (bio-)molécules dites de structure. Ainsi, un modèle simple de polyéthylène glycol (PEG) intégré dans un hydrogel a permis, selon la longueur du PEG d'obtenir un hydrogel biocompatible avec les propriétés rhéologiques voulues. C'est en effet la taille de la maille formée par la matrice qui permet à celle-ci dans le cas particulier des hydrogels d'absorber plus ou moins d'eau et de lui donner les propriétés rhéologiques voulues. En outre, en modifiant le nombre de groupements silylés introduits sur les (bio-)molécules de structure, il est possible de faire varier finement les propriétés rhéologiques de l'hydrogel quasiment indépendamment de la nature de ces (bio-)molécules de structure.

Un autre aspect de la présente invention concerne les techniques de purification des précurseurs d'hydrogels éventuellement fonctionnalisés. La première de ces techniques implique une série de précipitations, en particulier dans un solvant inerte tel que l'éther diéthylique. Cette technique présente en effet l'intérêt que, la majorité des précurseurs synthétiques fonctionnalisés d'hydrogels sont insolubles dans l'éther et ne sont pas réactifs dans ce solvant. Par une succession de lavages par l'éther et éventuellement de dissolutions dans un solvant adéquat, il est possible de proposer des précurseurs synthétiques fonctionnalisés d'hydrogels d'une pureté suffisante pour des molécules simples (peu coûteuses).

La présente invention propose en outre une méthode de purification par HPLC en phase inverse impliquant un choix particulier de substituants de l'atome de Si. En effet, l'utilisation d'un seul groupement réactif, tel qu'un halogène ou un groupement hydroxyle ou un précurseur d'hydroxyle, sur l'atome de Si permet une purification sur colonne HPLC inverse de la biomolécule silylée synthétisée, alors que les techniques selon l'art antérieur restent silencieuses quant à ce sujet. L'intérêt de pouvoir utiliser la HPLC en phase inverse, est de permettre l'obtention de molécules actives, telles que des biomolécules, avec un degré de pureté optimum, nécessaire dans le cadre d'une utilisation médicale.

Le choix des groupements portés sur l'atome de Si permet de pouvoir éviter dans la majorité des cas des réactions secondaires non voulues. Le potentiel de la présente invention réside donc également dans le fait qu'il semblerait que n'importe quelle biomolécule d'intérêt biologique (en particulier une séquence peptidique), puisse être introduite de façon contrôlée dans les hydrogels selon la présente invention pour leur conférer des propriétés biologiques.

Il a été de plus trouvé de manière totalement opportune des caractéristiques physico-chimiques originales d'un hydrogel de polyéthylène glycol bi-silylé permettant de manière très facile le greffage de biomolécules silylées. L'hydrogel de polyéthylène glycol bi-silylé ainsi formé est biocompatible, biodégradable, totalement synthétique, et facilement modulable en termes de taux d'hydratation (plus ou moins facilement imprégnable par des liquides par exemple aqueux). En outre il a été découvert que la matrice de l'hydrogel PEG(bi-silylé) peut être formée avec ou sans la présence d'eau, ce qui permet une flexibilité importante quant à l'utilisation de ce polymère (hydrogels déshydratés pouvant être hydratés par la suite). Néanmoins afin d'obtenir un hydrogel homogène en terme structurel (homogénéité de structure et d'hydratation), il est préférable de former l'hydrogel dans un milieu majoritairement aqueux. De plus, la possibilité de modifier la réactivité du groupement silyle en diminuant ou augmentant le nombre de substituants réactifs sur celui-ci (1, 2 ou 3 substituants réactifs), permet de finement contrôler le degré de réticulation de l'hydrogel et ainsi de pouvoir en contrôler finement sa rhéologie.

Un autre aspect selon la présente invention concerne l'insertion d'hydrogels liquides dans l'organisme avec polymérisation de ces hydrogels *in situ.* En effet, un problème récurrent des hydrogels liquides préparés préalablement est que ceux-ci sont difficilement injectables à cause de leur viscosité, occasionnant lors de leur administration (locale) au patient des douleurs plus ou moins aigües. La technique de WO2011089267, même si cela est évoqué dans ce document, n'est pas utilisable en tant que telle car la pureté des biomolécules silylées n'est pas suffisante pour une application médicale avec une injection sous forme liquide et prise *in situ.* Les méthodes de purification évoquées ci-dessus permettent de surmonter ce problème technique.

En effet, les hydrogels peuvent être préparés par simple dissolution des blocs hybrides dans un tampon phosphate puis incubation de la solution à 37°C, ce qui permet d'envisager leur injection *in vivo* avec prise *in situ.*

### Résumé de l'invention

L'objet de la présente invention concerne un procédé de fabrication d'un hydrogel comprenant les étapes de :
- a) polymérisation sol-gel d'au moins une molécule de formule (I) : 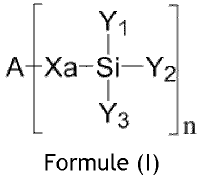 dans laquelle :
   - n est un nombre entier supérieur ou égal à 2
   - A est un polymère organique choisi parmi les protéines, les peptides tels que des dérivés du collagène, en particulier les séquences comprenant des répétitions de tripeptides Pro-Hyp-Gly ou Pro-Pro-Gly ou Asp-Pro-Gly ou Pro-Lys-Gly, des séquences peptidiques d'autoassemblage comme Arg-Ala-Asp-Ala , des oligoprolines, des oligoalanines, les polysaccharides, tels que l'acide hyaluronique et ses dérivés, les oligonucléotides, des polymères de C₁-C₆-alkylènes-glycols, ou de polyvinylpyrrolidone;
   - Xa est une liaison chimique ou un groupement espaceur représenté par un radical divalent dérivé d'une chaîne hydrocarbonée aliphatique saturée ou insaturée comportant de 1 à 10 atomes de carbone, dans laquelle sont éventuellement intercalés, un ou plusieurs chaînons structuraux choisis parmi le groupe arylène, ou les fragments -O-, -S-, - C(=O)-, -SO₂- ou -N(R₁)-, ladite chaîne étant non substituée ou substituée par un ou plusieurs radicaux choisis parmi les atomes d'halogène, les groupements hydroxyle, C₁-C₄ alkyle, benzyle et/ou phénéthyle ;
   - R₁ représente un atome d'hydrogène, un groupement hydrocarboné aliphatique comportant de 1 à 6 atomes de carbones, un benzyle ou un phénéthyle ;
   - Y₁, Y₂, Y₃, identiques ou différents, représentent chacun, indépendamment l'un de l'autre un atome d'hydrogène, d'halogène, un groupement -OR₂, un aryle ou une chaîne hydrocarbonée aliphatique saturée ou insaturée comportant de 1 à 6 atomes de carbone éventuellement substitués par un atome halogène, un groupement aryle ou hydroxyle ;
   - R₂ représente un atome d'hydrogène, un groupement aryle ou une chaîne hydrocarbonée aliphatique saturée ou insaturée comportant de 1 à 6 atomes de carbone ;
   - au moins deux groupements Xa tels que définis ci-dessus étant reliés à des points d'accroches différents sur A;
- b) mise en présence d'eau, optionnellement conjointement à l'étape a) ; préférentiellement la qualité de l'eau étant de qualité médicale, et
- c) récupération de l'hydrogel, caractérisé en ce que le procédé de polymérisation sol-gel est effectué à pH physiologique.

Le procédé de fabrication d'un hydrogel tel que défini ci-dessus peut en outre comprendre l'ajout conjointement ou successivement à l'étape a) au moins d'un type de molécule de formule (II) : 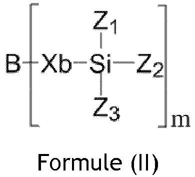 dans laquelle :
- m est un nombre entier supérieur ou égal à 1; préférentiellement m est inférieur à 10, plus préférentiellement inférieur à 5 ou 4, encore plus préférentiellement égal à 1 ;
- B est un principe actif choisi parmi un peptide, un oligopeptide, une protéine, tel que le collagène, un acide désoxyribonucléique, un acide ribonucléique, un polysaccharide, tel qu'une pectine, un chitosane, un acide hyaluronique, un polysaccharide polyarabinose et polygalactose, et un glycolipide;
- Xb est une liaison chimique ou un groupement espaceur représenté par un radical divalent dérivé d'une chaîne hydrocarbonée aliphatique saturée ou insaturée comportant de 1 à 10 atomes de carbone, dans laquelle sont éventuellement intercalés, un ou plusieurs chaînons structuraux choisi parmi le groupe arylène, ou les fragments -O-, -S-, -C(=O)-, -SO₂- ou -N(R₃)-, ladite chaîne étant non substituée ou substituée par un ou plusieurs radicaux choisis parmi les atomes d'halogène, les groupements hydroxyle, C₁-C₄ alkyle, benzyle et/ou phénéthyle ;
- R₃ représente un atome d'hydrogène, un groupement hydrocarboné aliphatique comportant de 1 à 6 atomes de carbone, un benzyle ou un phénéthyle ;
- Z₁, Z₂, Z₃, identiques ou différents, représentent chacun, indépendamment l'un de l'autre un atome d'hydrogène, d'halogène, un groupement -OR₄, un aryle ou une chaîne hydrocarbonée aliphatique saturée ou insaturée comportant de 1 à 6 atomes de carbone éventuellement substitués par un atome halogène, un groupement aryle ou hydroxyle ;
- R₄ représente un atome d'hydrogène, un groupement aryle ou une chaîne hydrocarbonée aliphatique saturée ou insaturée comportant de 1 à 6 atomes de carbone ; et
- avec préférentiellement un seul des groupements Z₁, Z₂, ou Z₃ comme atome d'halogène ou groupement OR₄.

L'objet de la présente invention, concerne donc un hydrogel susceptible d'être obtenu par le procédé tel que défini présentement.

L'objet de la présente invention concerne en outre un hydrogel selon la présente invention pour son utilisation à des fins thérapeutiques et/ou chirurgicales, préférentiellement caractérisé en ce que ledit hydrogel permet la délivrance et/ou le transport de molécules actives, ou pour son utilisation *in vivo* en ingénierie tissulaire

Ainsi, la présente invention concerne un hydrogel susceptible d'être obtenu par le procédé selon la présente invention pour son utilisation telle que définie ci-dessus, caractérisé en ce que la polymérisation dudit hydrogel est réalisée *in situ* dans un organisme vivant, c'est à dire un animal tel qu'un mammifère comme l'Homme, suite à la coulée ou l'injection, préférentiellement indolore ou peu douloureuse, des molécules de formule (I) et éventuellement (II) telles que définies ci-dessus.

Un autre objet de la présente invention concerne l'utilisation *in vitro* d'un hydrogel selon la présente invention en ingénierie tissulaire.

De plus, la présente description concerne un procédé de purification d'un produit de formule
(I) ou (II) tel que défini présentement, caractérisé en ce que ledit procédé comprend les étapes suivantes:
   - a1) éventuelle solubilisation du produit de formule (I) ou (II) à purifier dans un solvant ;
   - b1) précipitation du produit de formule (I) ou (II) à purifier, éventuellement en solution selon l'étape a1), dans un liquide de suspension, i.e. dans lequel les produits (I) ou (II) ne sont pas solubles ;
   - c1) filtration du solide obtenu à l'étape (b1) ;
   - d1) répétition au moins une fois des étapes a1) le cas échéant, b1) et c1) ; et
   - e1) récupération du produit de formule (I) ou (II) purifié.

L'objet de la présente description concerne de plus un procédé de purification d'un produit de formule (II) tel que défini présentement dans lesquels un seul des groupements Z₁, Z₂, Z₃ est un atome d'halogène ou un groupement -OR₃, caractérisé en ce que ledit procédé comprend les étapes suivantes:
- a2) passage du produit de formule (II) à purifier sur une colonne de chromatographie en phase liquide, i.e. dans un solvant d'élution, préférentiellement en phase inverse ;
- b2) évaporation du solvant d'élution de l'étape a2), éventuellement par lyophilisation ; et
- c2) récupération du produit de formule (II) purifié.

Ainsi, la présente description concerne également un produit de formule (I) ou (II) tel que défini présentement, susceptible d'être obtenu par le procédé de purification de l'invention caractérisé en ce que ledit produit présente une pureté d'au moins 98% en masse vis-à-vis du poids total de produit, préférentiellement supérieure à 99% en masse.

### Définitions

### « Hydrogel »

Un « hydrogel » est un type de matériau comportant une composante liquide aqueuse et une composante solide. Structurellement, il est composé d'une matrice de chaînes polymères, gonflée par un fluide comprenant de l'eau, ce fluide, préférentiellement constitué essentiellement d'eau, étant préférentiellement en une proportion égale ou supérieure à 40% en poids dudit hydrogel, plus préférentiellement comprise entre 40 et 99% en poids, entre 50 et 98%, entre 60 et 97%, entre 70 et 96% ou encore entre 80% et 95%. De manière préférée ce fluide comprend au moins 95% d'eau en poids, voire 100% d'eau. Ce fluide peut en outre être un liquide d'origine biologique. La teneur en eau d'un hydrogel détermine également en grande partie ses caractéristiques physico-chimiques. Il est possible de retrouver ces hydrogels également pour diverses applications biomédicales notamment dans la libération de médicaments et dans le traitement de brûlures cutanées.

### « Polymère pouvant être réticulé » ou « polymère réticulable »

Les hydrogels selon l'invention sont constitués de chaînes de polymères liées entre elles par des liaisons covalentes. Un polymère réticulé comprend des noeuds avec au moins 3 liaisons chimiques, préférentiellement covalentes. Cette réticulation permet aux hydrogels selon la présente invention de fournir un caractère dit "permanent" aux noeuds de réticulation car l'atome de silicium tétravalent génère 4 liaisons covalentes. Néanmoins, de par la double nature des hydrogels selon la présente invention (organique et inorganique), il est possible d'obtenir des propriétés remarquables : une rigidité partielle et une capacité à donner une réponse élastique à un stress mécanique. La nature organique permet une haute biocompatibilité et une non-toxicité des hydrogels produits. En outre, les hydrogels selon la présente invention ont un degré de flexibilité très similaire à celui des tissus naturels (induit par leur importante teneur en eau). Le terme « réticulable » fait donc préférentiellement référence ici à la capacité des atomes de silicium à générer au plus 3 liaisons covalentes avec au plus 3 groupements comprenant un atome de Si (tel que Si-OH).

### « Polymérisation sol-gel »

Le procédé sol-gel permet de fabriquer un polymère inorganique par des réactions chimiques simples à une température proche de la température ambiante (en réalité applicable à des gammes de températures allant de 0°C à 150°C, préférentiellement entre 20°C et 70°C, plus préférentiellement entre 35°C et 40°C). Typiquement, la synthèse est effectuée à partir d'alcoxysilanes ou de silanols de formule Si(OR)ₙ où R est un groupement organique alkyle CₙH₂ₙ₊₁ ou un hydrogène.

Un des intérêts de ce procédé est que ces précurseurs sont soit liquides soit solides, dans ce cas ils sont, pour la plupart, solubles dans des solvants usuels. Il est donc possible de préparer des mélanges homogènes de monomères (précurseurs) ou oligomères.

Les réactions chimiques simples à la base du procédé sont déclenchées lorsque les précurseurs sont mis en présence d'eau : l'hydrolyse des groupements typiquement alcoxy (ou halogène le cas échéant) intervient tout d'abord, puis la condensation des produits hydrolysés conduit à la gélification du système, formant ainsi l'hydrogel.

### « Polymère organique de structure »

Par « polymère organique de structure », il est compris selon la présente invention un polymère de nature organique, donc hydrocarboné, permettant de structurer l'hydrogel sous forme d'une matrice polymérique. Ceci est réalisé facilement à partir du moment où les monomères composant ledit polymère organique de structure sont contrôlés en terme de structure chimique et de pureté ; i.e. par les techniques usuelles de chimie de synthèse ou de purification. En effet, en contrôlant la nature des monomères impliqués dans la polymérisation de l'hydrogel, il est possible de faire varier les caractéristiques physico-chimiques et rhéologiques du polymère obtenu *in fine.*

### « Origine synthétique »

Les matières premières utilisées pour la production des matériaux synthétiques sont bien sûr issues de la Nature, comme le pétrole. Cependant, les matériaux synthétiques créés par l'Homme à partir de procédés chimiques, les différencient des autres matériaux. Selon la présente invention, l'expression « origine synthétique » implique que le produit considéré a été modifié au moins une fois par un procédé chimique mis au point par l'Homme.

### « Liaison chimique »

On appelle « liaison chimique » toute interaction attractive qui maintient au moins deux atomes à courte distance l'un de l'autre. Cette interaction peut être directionnelle comme la liaison entre deux atomes au sein d'une molécule, ou non directionnelle comme l'interaction électrostatique qui maintient les ions d'un cristal ionique en contact. Elle peut être forte comme les deux précédents exemples, ou faible comme les interactions de van der Waals qui sont de nature dipolaire.

### « Groupement espaceur »

On appelle « groupement espaceur » selon la présente invention, un fragment comprenant au moins un atome. Préférentiellement, le groupement espaceur contient au moins un atome de carbone. Avantageusement le groupe espaceur permet d'éloigner au sein d'une même molécule, deux groupements chimiques, de diminuer la gêne stérique entre le groupement A (ou B) et l'atome de Si. Plus avantageusement, le groupement espaceur permet au groupement silylé de réagir avec une gêne limitée du fragment A (ou B) ou au groupement A (ou B) d'interagir et de conserver ses propriétés biologiques, avec une gêne limitée du fragment contenant le Si. En outre le groupement espaceur permet une liaison stable entre le fragment A (ou B) et Si, tout en permettant au fragment silicate de réagir. Il est donc clair que le groupement espaceur ne peut pas être considéré comme un élément constitutif du fragment A (ou B), comme par exemple un résidu d'acide aminé si A (ou B) est un fragment peptidique).

Avantageusement, le groupement espaceur comprend, ou consiste en, une chaîne hydrocarbonée aliphatique saturée ou insaturée, préférentiellement comprise entre 1 et 10 atomes de carbone, plus préférentiellement entre 2 et 5 atomes de carbone. De manière préférée, le groupement espaceur est une chaîne hydrocarbonée aliphatique saturée.

Le groupement espaceur (en particulier lorsqu'il s'agit d'une une chaîne hydrocarbonée aliphatique saturée ou insaturée telle que décrite ci-dessus) peut en outre inclure des hétéroatomes, en particulier choisis parmi N, O, S, ou encore P, et peut en plus être substitué, en particulier par des atomes d'halogènes, ou par des groupements hydroxyles, aryles, alkyles en C₁-C₄, sulfates, amines, ou encore phosphates. Toutefois, si des hétéroatomes sont présents dans le groupement espaceur, préférablement ces hétéroatomes ne sont pas directement reliés à Si.

Préférablement, le groupement espaceur est un fragment alkyle en C₁-C₄ linéaire ou ramifié. Plus préférablement, le groupement espaceur comprend un fragment -(CH₂)₂-, - (CH₂)₃-, -(CH₂)₄-. De manière plus préférée encore, le groupement espaceur comprend le fragment -(CH₂)₃-.

### « Radical divalent dérivé »

Selon la présente invention, l'expression « radical divalent dérivé » concerne un élément ayant une valence de deux. La valence est le nombre de liaisons chimiques formées, qui peuvent être des liaisons covalentes, polaires, ou ioniques. Le terme « dérivé » fait simplement référence aux liaisons chimiques formées pour incorporer ledit radical dans la structure.

### « Chaîne hydrocarbonée aliphatique saturée ou insaturée »

Par « chaîne hydrocarbonée aliphatique saturée ou insaturée », il est compris des fragments de type alkyle en C₁-C₁₀, alcène en C₂-C₁₀ ou alcyne en C₀₂-C₁₀, préférentiellement ces chaines sont linéaires ou ramifiées.

### « Alkyle en C₁-C₁₀ »

Dans la présente invention, il est compris par « alkyle en C₁-C₁₀ », ou encore « alkyle de 1 à 10 atomes de carbone », un groupe aliphatique saturé, linéaire, ramifié, voire cyclique, comportant de 1 à 10 atomes de carbone, comme par exemple un groupement méthyle, éthyle, isopropyle, tertio-butyle, n-pentyle, cyclopropyle, cyclohexyle, etc.

### « Alcène en C₂-C₁₀ »

Par groupement « alcène en C₂-C₁₀ », ou encore « alcène de 2 à 10 atomes de carbone », on entend au sens de la présente invention, un groupe aliphatique mono- ou poly-insaturé, linéaire, ramifié ou cyclique comprenant de 2 à 10 atomes de carbone. Un groupe alcène selon l'invention comprend, de préférence, une ou plusieurs insaturations éthyléniques. A titre d'exemple, on peut citer les groupes éthylènes, propylène, propyl-2-ène ou propyl-3-ène, butylène, cyclobutène etc.

### « Alcyne en C₂-C₁₀ »

Par groupement « alcyne en C₂-C₁₀ », ou encore « alcyne de 2 à 10 atomes de carbone », on entend au sens de la présente invention, un groupe aliphatique, linéaire, ramifié ou cyclique comprenant de 2 à 10 atomes de carbone et au moins une double insaturation, c'est-à-dire une triple liaison entre deux atomes de carbones. Un groupe alcyne selon l'invention comprend, de préférence, une ou plusieurs doubles insaturations. A titre d'exemple, on peut citer les groupes acétylène, propyne, butyne, etc.

### « Aryle »

Par groupement « aryle », on entend un groupement aromatique, comportant de préférence de 5 à 10 atomes de carbone, comprenant un ou plusieurs cycles et comprenant éventuellement un ou plusieurs hétéroatome(s) en particulier un oxygène, un azote ou un soufre, comme par exemple un groupement phényle, furane, indole, pyridine, naphtalène, etc.

### « Arylène »

Un groupement « arylène » représente un substituant d'un composé organique dérivé d'un fragment aryle dans lequel au moins un atome d'hydrogène a été retiré de deux carbones inclus dans l'aryle. Préférentiellement, il s'agit d'un groupement phénéthyle.

### « Phénéthyle »

Le terme phénéthyle représente le fragment :

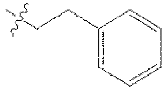

### « Halogène »

Le terme halogène désigne les éléments chimiques de la 17^{e} colonne du tableau périodique, anciennement appelé groupe VII ou VIIA. Ces éléments chimiques sont préférentiellement : le fluor, le chlore, le brome et l'iode.

### « Hydroxyle »

Le terme hydroxyle selon la présente invention concerne le fragment -OH, et éventuellement ses sels, par exemple de sodium ou potassium.
*« Xa reliés* à *des points d'accroches différents sur A* »;

Par l'expression « Xa reliés à des points d'accroches différents sur A », il est compris selon la présente invention que les groupements Xa ne sont pas reliés au même atome appartenant au polymère A. De manière préférée, les points d'accroches (i.e. les atomes d'accroche) sont positionnés de manière la plus éloignée possible sur le polymère A. Une manière pour évaluer cet éloignement peut être simplement de compter le nombre d'atomes entre les deux points d'accroche sur le polymère A. En effet, les propriétés physico-chimiques et rhéologiques sont plus facilement contrôlables lorsque l'entropie des polymères A est diminuée, ce qui se fait en principe en fixant les extrémités des chaînes de polymère.

### « Récupération »

Par « récupération », il est compris selon la présente invention que les produits obtenus sont extraits selon les techniques communes de l'art, i.e. par l'intermédiaire d'un lavage biphasique comprenant par exemple un solvant organique et de l'eau ; alternativement, il est possible de récupérer les produits par suspension dans le liquide qui les contient, puis de les filtrer. Une autre façon de récupérer les produits, peut être tout simplement d'évaporer ou lyophiliser le solvant qui les contient. Cette phase de récupération peut en outre contenir une purification par lavage ou passage sur colonne chromatographique, le cas échéant.

### « Eau de qualité médicale »

Par « eau de qualité médicale », il est compris selon la présente invention que l'eau est de qualité ultra pure communément utilisée dans le milieu médical, éventuellement avec des adjuvants permettant de retrouver les conditions physiologiques de l'organisme humain, tel que des sels comme NaCl, KCl, CaCl₂, MgCl₂, etc. ; de tampon pH tel que des tampons phosphates pH 7,4, etc. ; ou encore des sucres tels que du glucose, du mannitol.

### « Principe actif »

La définition générale d'un principe actif, est : le principe actif est la molécule qui dans un médicament possède un effet thérapeutique. Dans le contexte de la présente invention, qui concerne les hydrogels, le principe actif est la molécule présentant des caractéristiques physico-chimiques ou biologiques distinctives du polymère qui la porte. Par exemple, le principe actif peut être un médicament connu, une molécule biologique telle qu'une séquence peptidique permettant l'accroche de cellules biologiques, ou un colorant ou toute autre molécule présentant une ou des activités biologiques ou physico-chimiques. Le principe actif peut être d'origine naturelle ou synthétique.

De manière avantageuse, l'hydrogel selon la présente invention comprend un principe actif à activité antimicrobienne, antibiotique et/ou antifongique.

Préférentiellement, le principe actif est une biomolécule, par exemple choisie parmi un peptide, une protéine, un glycopeptide, une glycoprotéine, un acide déoxyribonucléique, un acide ribonucléique, une pectine, un chitosane, un acide hyaluronique, un saccharide, un oligosaccharide, un lipide, un glycolipide, ou leurs dérivés.

De manière avantageuse le principe actif est choisi parmi des molécules favorisant l'adhésion cellulaire, telles que des peptides contenant ou consistant en la séquence ArgGlyAsp, en particulier, le peptide H-GlyArgGlyAspSerPro-OH (Seq ID 1), des molécules favorisant la cicatrisation, telles que des peptides contenant ou consistant en la séquence H-GluGlyLeuGluProGly-OH (Seq ID 2), des molécules favorisant la production de matrice extracellulaire telles que des peptides contenant ou consistant en H-ValGlyValAlaProGly-OH (Seq ID 3) ou des molécules antibactériennes, telles que des peptides contenant ou consistant en H-AhxArgArg-NH₂, des molécules (e.g. peptides) antidouleurs, des molécules

(e.g. peptides) qui favorisent la coagulation du sang, des molécules (e.g. peptides) anticoagulantes, des molécules (e.g. peptides) antiprolifératives, ou un mélange de plusieurs de ces (bio-)molécules.

De manière avantageuse le principe actif peut être un colorant, un fluorophore ou un marqueur choisi parmi les composés suivants : fluorescéine, sel sodique de fluorescéine, 4',5'-Bis[*N*,*N-*bis(carboxyméthyl)-aminométhyl]fluorescéine, acide 6-[fluorescéine-5(6)-carboxamido]hexanoïque, acide 6-[fluorescéine-5(6)-carboxamido]hexanoïque, ester N-hydroxysuccinimide de fluorescéin-5(6)-isothiocyanate, fluorescéin-α-D-N-acétylneuraminide-polyacryl-amide, amidite de fluorescéine, fluorescéine-di(β-D-galactopyranoside), fluorescéin-di-(B-D-glucopyranoside), diacétate de fluorescéine, diacétate fluorescéin-5(6)-isothiocyanate, diacétate de fluorescéin-5-maleimide, diacétate de fluorescéin-6-isothiocyanate, dibutyrate de fluorescéine, fluorescéine dilaurate, sel de diphosphate de fluorescéine triammonium, acide fluorescéin-hyaluronique, isothiocyanate de fluorescéine- Dextran 500000-Conjugué, isomère I de l'isothiocyanate de fluorescéine, isothiocyanate de fluorescéine-dextran, acétate de mercure-fluorescéine, chlorhydrate de mono-p-guanidinobenzoate-fluorescéine, *O*,*O'*-diacrylate de fluorescéine, fluorescéine *O*,*O'*-diméthacrylate, fluorescéine *o*-acrylate, fluorescéine *O*-méthacrylate, ester N-hydroxysuccinimide de fluorescéine, fluorescéine-5-thiosemicarbazide, fluorescéin-α-D-galactosamine polyacrylamide, fluorescéin-α-D-mannopyranoside-polyacrylamide, 4(5)-(iodoacétamido)-fluorescéine, 5-(Bromométhyl)fluorescéine, 5-(Iodoacétamido)fluorescéine, diacétate de l'ester N-succinimidyl-5-Carboxy-fluorescéine, diacétate de l'ester N-succinimidyl-6-Carboxy- fluorescéine, Aminophényl-fluorescéine, Biotin-4- fluorescéine, hydroxyphényl-fluorescéine, MTS-4- fluorescéine, chlorhydrate de poly(fluorescéine-isothiocyanate allylamine), poly(fluorescéine-O-acrylate), poly(fluorescéine-O-méthacrylate), acétate de PPHT-fluorescéine, chlorhydrate de 5-([4,6-dichlorotriazin-2-yl]amino) fluorescéine, chlorhydrate de 6-([4,6-dichlorotriazin-2-yl]amino) fluorescéine, poly[(méthylméthacrylate)-*co*-(fluorescéine-*O*-méthacrylate)], poly[méthylméthacrylate-co-(fluorescéine *O*-acrylate)], 5(6)-(Biotinamidohexanoylamido)pentylthioureidylfluorescéine, N-(5- fluorescéinyl)maléimide, sel disodique de Mercure-dibromo-fluorescéine, fluorescéin-di-[méthylène-N-méthylglycine], sel disodique de 2',4',5',7'-tétrakis-(acétoxymercuro)-fluoroscéine, l'érythrosine B, l'éthyl éosine, 5-carboxy fluorescéine, ester N-succinimidyl de 5-carboxy fluorescéine, rhodamine B octadécyl, ester N-hydroxysuccinimide de 6-Carboxy-fluorescéine, dibenzyl fluorescéine, rhodol, 6-amino fluorescéine, rhodamine 6G, la rhodamine B ou encore la rhodamine 123. Ces colorants, fluorophores et/ou marqueurs peuvent être incorporés dans l'hydrogel avec une biomolécule ou un mélange de plusieurs biomolécules.

Le terme « biomolécule » selon la présente invention concerne une molécule susceptible d'être trouvée dans le milieu biologique, i.e. synthétisée par un organisme vivant. Cette définition comprend également les analogues fonctionnels de ces molécules (qui deviennent donc des molécules de synthèse). En effet, il est par exemple commun dans l'art de modifier la structure des biomolécules afin d'uniquement en garder la région active ou le site actif, ou d'ajouter des groupements protecteurs des fonctions réactives, empêchant ainsi les réactions secondaires, par exemple.

Ces groupements protecteurs et leur utilisation sont décrits dans des ouvrages tels que par exemple Greene, "Protective Groups in Organic Synthesis", Wiley, New York, 2007 4e édition ; Harrison et al. "Compendium of Synthetic Organic Methods", Vol. 1 à 8 (J. Wiley Et sons, 1971 to 1996).

### « Activité antimicrobienne »

Une « activité antimicrobienne » selon la présente invention est la définition générique telle que comprise par l'homme du métier, c'est-à-dire un effet relatif à un agent antimicrobien. Un (agent) antimicrobien est une substance qui tue, ralentit la croissance ou bloque la croissance d'un ou de plusieurs microbes. Par « croissance » il est compris dans le cadre de la présente invention toute opération cellulaire permettant un accroissement volumétrique de la cellule, une division cellulaire ou une reproduction cellulaire. Un microbe dans le cadre de la présente invention concerne tout organisme unicellulaire ou pluricellulaire pathogène ou parasitaire d'autres organismes vivants comme les humains.

Par exemple, les antimicrobiens peuvent être généralement choisis parmi les différentes familles suivantes : les bêtalactamines, les céphalosphorines, la fosfomycine, les glycopeptides, les polymyxines, les gramicidines, la tyrocidine, les aminosides, les macrolides, les lincosamides, les synergistines, les phénicolés, les tétracyclines, l'acide fusidique, les io oxazolidinones, les rifamycines, les quinolones, les fluoroquinolones, les produits nitrés, les sulfamides, la triméthoprime, et leurs mélanges. De manière plus spécifique les antimicrobiens peuvent être choisis parmi la nystatine, le nitrate de miconazole, l'acide lauryl-oxypropyl-β-aminobutyrique, l'amphotéricine B, l'acide undécylénique, le chloroquinaldol, le nitrate d'éconazole, la natamycine, le cloprothiazole, le clotrimazole, le tolnaftate, la lucensomycine, la tétracycline, l'érythromycine, les pénicillines, l'oxacilline, la cloxaciline, l'ampicilline, l'amoxicilline, la bacampicilline, la métampicilline, la pivampicilline, l'azlocilline, la mezlocilline, la pipéracilline, la ticarcilline, le pivmécillinam, le sulbactam, le tazobactam, l'imipénème, la céfalexine, le céfadroxil, le céfaclor, la céfatrizine, la céfalotine, la céfapyrine, la céfazoline, la céfoxitine, le céfamandole, le céfotétan, le céfuroxime, le céfotaxime, le céfsulodine, le céfopérazone, le céfotiam, la céftazidime, le céftriaxone, le céfixime, le céfpodoxime, le céfépime, le latamoxef, l'aztréonam, la vancomycine, la vancocine, la téicopianine, la polymyxine B, la colistine, la bacitracine, la tyrothricine, la streptomycine, la kanamycine, la tobramycine, l'amikacine, la sisomicine, la dibékacine, la nétilmicine, la spectinomycine, la spiramycine, l'érythromycine, la josamycine, la roxithromycine, la clarithromycine, l'azithromycine, la lincomycine, la clindamycine, la virginiamycine, la pristinamycine, la dalfopristine-quinupristine, le chloramphénicol, le thiamphénicol, la tétracycline, la doxycycline, la minocycline, l'acide fusidique, la linézolide, la rifamycine, la rifampicine, l'acide nalidixique, l'acide oxolinique, l'acide pipémidique, la fluméquine, la péfloxacine, la norfloxacine, l'ofloxacine, la ciprofloxacine, l'enoxacine, la sparfloxacine, la lévofloxacine, la moxifloxacine, la nitroxoline, le tilboquinol, la nitrofurantoïne, la nifuroxazide, la métronidazole, l'ornidazole, la sulfadiazine, le sulfaméthisol, le triméthoprime, l'isoniazide et leurs dérivés et mélanges.

Préférentiellement, les antimicrobiens greffés ont un mode d"action de contact.

En outre, toutes ces molécules antimicrobiennes présentent des fonctions chimiques plus ou moins réactives. Ainsi, l'homme du métier est tout à fait capable d'adapter l'objet de la présente invention afin d'utiliser l'une de ces fonctions comme point d'attache au fragment Xb.

### « Activité antibiotique »

Une « activité antibiotique » (équivalent au terme « antibactérien ») selon la présente invention est la définition générique telle que comprise par l'homme du métier, c'est-à-dire un effet relatif à un agent antibiotique. Un (agent) antibiotique est une substance est une substance qui tue, ralentit la croissance ou bloque la croissance d'une ou de plusieurs bactéries. Par « croissance » il est compris dans le cadre de la présente invention toute opération cellulaire permettant un accroissement volumétrique de la cellule (bactérie), une division cellulaire (de la bactérie) ou une reproduction cellulaire (de la bactérie).

Par exemple les antibiotiques peuvent être généralement choisis parmi les différentes familles suivantes : les béta-lactames, les monobactames, les pénicillines, les inhibiteurs de beta lactamases, les aminoglycosides, les glycylcycline, les tétracyclines, les quinolones, les glycopeptides, les lipopeptides, les macrolides, les ketolides, les lincosamides, les streptogramines, les oxazolidinones, les polymyxines.

De manière plus spécifique les antibiotiques peuvent être choisis parmi l'amikacine, la gentamycine, la tobramycine, l'imipénème, le méropénème, l'ertapénème, le composé connu sous l'appellation PZ-601, la céfazoline, la céfépime, la céfotaxime, la céfoxitine, la ceftaroline, la ceftazidime, la ceftibiprole, la ceftriaxone, la céfuroxime, la céphalexine, l'aztréoname, l'amoxicilline, la clavulanate, l'ampicilline, le sulbactame, l'oxacilline, la pipéracilline, le tazobactame, la ticarcilline, la pénicilline, la doxycycline, la minocycline, la tétracycline, la tigécycline, la ciprofloxacine, la gatifloxacine, la grépafloxacine, la lévofloxacine, la moxifloxacine, l'ofloxacine, l'azithromycine, la clarithromycine, la roxythromycine, la télithromycine, la colistine, la polymyxine B, la fosfomycine, le triméthoprim et le sulfaméthoxazole.

On peut encore citer comme exemples d'agents antibiotiques les alcools, les polyols en C2-C8, les acétate, benzoate et diacétate d'aluminium, des conservateurs tels que le chlorure de benzalkonium, le chlorure de cétrimonium, la chlorhexidine, le climbazole, des citrates, l'oxyde et le sulfate d'argent, des acides tel que l'acide borique, l'acide usnique, l'acide pyroglutamique et dérivés, les acétate, borate, salicylate et sulfate de zinc, des peptides antimicrobiens tels que les béta-défensines, et leurs mélanges.

Préférentiellement, les antibiotiques greffés ont un mode d"action de contact.

Toutes ces molécules antibiotiques présentent des fonctions chimiques plus ou moins réactives. Ainsi, l'homme du métier est tout à fait capable d'adapter l'objet de la présente invention afin d'utiliser l'une de ces fonctions comme point d'attache au fragment Xb.

### « Activité antifongique »

Une « activité antifongique » selon la présente invention est la définition générique telle que comprise par l'homme du métier, c'est-à-dire un effet relatif à un agent antifongique. Un (agent) antifongique est une substance qui tue, ralentit la croissance ou bloque la croissance d'un ou de plusieurs champignons. Par « croissance » il est compris dans le cadre de la présente invention toute opération cellulaire permettant un accroissement volumétrique de la cellule (champignon), une division cellulaire (du champignon) ou une reproduction cellulaire (du champignon).

Des exemples d'antifongiques peuvent en outre être choisis parmi les différentes familles suivantes : les polyènes, les imidazoles, les triazolés, les analogues nucléosidiques, les allylamines, les échinocandines, les sordarines, les morpholines, la griséofulvine, la ciclopiroxolamine, le sulfure de sélénium, et leurs mélanges.

Plus préférablement, l'agent antifongique est choisi parmi la nystatine, l'amphotéricine B, le kétoconazole, l'éconazole, le miconazole, le clotrimazole, le fluconazole, l'itraconazole, le voriconazole, le posaconazole, la 5-fluorocytosine, la naftafine, la terbinafine, la caspofongine, l'amorlfine, et leurs dérivés et mélanges.

Préférentiellement, les antifongiques greffés ont un mode d"action de contact.

Toutes ces molécules antifongiques présentent des fonctions chimiques plus ou moins réactives. Ainsi, l'homme du métier est tout à fait capable d'adapter l'objet de la présente invention afin d'utiliser l'une de ces fonctions comme point d'attache au fragment Xb.

### « Principe actif naturel »

Un principe actif naturel, telle qu'une biomolécule naturelle, est un principe actif trouvé dans l'environnement sans intervention humaine directe (hormis son extraction/isolement)

### « Principe actif synthétique »

Un principe actif synthétique est un principe actif qui n'est pas trouvé dans l'environnement sans intervention humaine directe (hormis son extraction/isolement). Par exemple, un principe actif tel qu'un peptide synthétique peut être une séquence d'un peptide naturel dans lequel au moins un acide aminé naturel a été remplacé par un autre, naturel ou synthétique.

### « Peptide » et « protéine »

Les termes « peptide » (équivalent au terme « oligopeptide ») et « protéine » doivent être compris comme des polymères d'acides aminés, lesdits acides aminés étant reliés entre eux par une liaison peptidique et/ou pseudopeptidique. Un peptide contient généralement entre 2 et 80 à 100 acides aminés, la limite supérieure n'étant pas clairement définie. Au-delà de cette limite supérieure, on parlera plutôt de protéines. Préférentiellement le principe actif peptidique selon la présente invention contient entre 2 et 80 acides aminés, plus préférablement entre 3 et 40, et encore plus préférablement entre 4 et 20.

Les peptides ou protéines peuvent être extraites d'un milieu biologique ou fabriquées de manière synthétique. Les techniques de synthèse peptidique sont décrites dans Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002.

### « Acide aminé »

L'expression « acide aminé » doit être comprise comme toute molécule présentant au moins un acide carboxylique, au moins une amine et au moins un carbone reliant cette amine et cet acide carboxylique. Préférentiellement, les acides aminés pouvant être utilisés dans le cadre de la présente invention sont les acides aminés dits « naturels » et/ou les acides aminés synthétiques tels que défini ci-dessous. Préférentiellement les acides aminés de la présente invention sont de configuration L.

### « Acide aminé naturel »

L'expression « acide aminé naturel » représente entre autres les acides aminés suivants : la glycine (Gly), l'alanine (Ala), la valine (Val), la leucine (Leu), l'isoleucine (Ile), la sérine (Ser), la thréonine (Thr), la phénylalanine (Phe), la tyrosine (Tyr), le tryptophane (Trp), la cystéine (Cys), la méthionine (Met), la proline (Pro), l'acide aspartique (Asp), l'asparagine (Asn), la glutamine (Gln), l'acide glutamique (Glu), l'histidine (His), l'arginine (Arg) et la lysine (Lys). Les acides aminés naturels préférés selon la présente invention sont les acides aminés de la série L.

### « Acide aminé synthétique »

Par acide aminé synthétique, il est compris tous les acides aminés non « codés » et non naturels tels que définis ci-dessus.

### « Glucide »

Le terme "glucide" comprend les monosaccharides et polysaccharides.

Un monosaccharide, ou ose, est un polymère de carbones hydraté, lesquels carbones sont reliés entre eux par une liaison C-C. Il existe deux types d'ose : les aldoses et les cétoses. Les monosaccharides sont en outre « cyclisables » par l'intermédiaire d'une fonction hémiacétal. Les monosaccharides préférés selon la présente invention sont les monosaccharides de la série D. Les monosaccharides sont classés par nombre de carbones. Par exemples les monosaccharides à 6 carbones sont les hexoses de formule C₆H₁₂O₆ et peuvent être l'allose, l'altrose, le glucose, le mannose, le gulose, l'idose, le galactose ou le talose. Les monosaccharides à 5 carbones sont les pentoses de formule C₅H₁₀O₅ et peuvent être le ribose, l'arabinose, le xylose, ou le lyxose.

Un polysaccharide concerne un polymère fait de monosaccharides (préférentiellement de la série D) joints par des liaisons glycosidiques. Des exemples de polysaccharides sont la cellulose et ses dérivés, la pectine, le chitosane, ou encore l'acide hyaluronique. Les dérivés de cellulose comprennent l'hydroxypropylméthylcellulose (HPMC), l'hydroxyéthylcellulose (HEC), l'hydroxypropylcellulose (HPC), carboxyméthylcellulose (CMC).

Les glucides naturels ou synthétiques sont compris dans cette définition.

### « Glycopeptide »

Un glycopeptide est un peptide relié à un glucide par l'intermédiaire d'au moins une liaison chimique.

### « Glycoprotéine »

Une glycoprotéine est une protéine reliée à un glucide par l'intermédiaire d'au moins une liaison chimique.

### « Acide déoxyribonucléique »

L'acide désoxyribonucléique, ou ADN, est une macromolécule biologique présente dans toutes les cellules ainsi que chez de nombreux virus. L'ADN contient toute l'information génétique, appelée génotype, permettant le développement et le fonctionnement des êtres vivants.

### « Acide ribonucléique »

L'acide ribonucléique (ARN) est une molécule biologique présente chez pratiquement tous les êtres vivants, et aussi chez certains virus. L'ARN est une molécule très proche chimiquement de l'ADN et il est d'ailleurs en général synthétisé dans les cellules à partir d'une matrice d'ADN dont il est une copie. Les cellules vivantes utilisent en particulier l'ARN comme un support intermédiaire des gènes pour synthétiser les protéines dont elles ont besoin.

### « Pectine »

Les pectines sont des polysaccharides caractérisés par un squelette d'acide α-D-galacturonique et de faibles quantités de α-L-rhamnose plus ou moins ramifiés.

### « Chitosane »

Le chitosane ou chitosan est un polyoside composé de la distribution aléatoire de D-glucosamine liée en β-(1-4) (unité désacétylée) et de N-acétyl-D-glucosamine (unité acétylée).

### « Acide hyaluronique »

Les acides hyaluroniques sont des polymères de disaccharides eux-mêmes composés d'acide D-glucuronique et de D-N-acétylglucosamine, liés entre eux par des liaisons glycosidiques alternées □□1,4 et □□1,3. Les polymères de cette unité récurrente peuvent avoir une taille moyenne entre 400 et 10⁷ Da *in vivo.* Dans le cadre de la présente invention, les polymères de cette unité récurrente peuvent avoir une taille moyenne entre 1000 et 7.10⁶ Da, préférentiellement entre 1500 et 5.10⁶ Da, par exemple entre 2000 et 9000 Da ou entre 10⁴ et 4.10⁶, telle qu'entre 150000 et 500000 ou encore entre 2.10⁶ et 3.10⁶. Par exemple dans le cadre de la présente invention, les polymères de cette unité récurrente peuvent avoir une taille moyenne d'environ 6400 Da, 2.10⁵ Da ou encore 2,6.10⁶ Da.

### « Lipide »

Les lipides comprennent plusieurs classes de molécules comprenant les acides gras, les glycérides, les phosphoglycérides, les sphingolipides, les stérols, les prénols.

Les acides gras sont des acides carboxyliques à chaîne aliphatique. Les glycérides sont constitués d'un résidu de glycérol estérifié par un, deux ou trois acides gras, ce qu'on appelle respectivement monoglycérides, diglycérides et triglycérides. Les phosphoglycérides sont des phospholipides constitués de deux résidus d'acides gras estérifiant un résidu glycérol lui-même estérifié par un résidu phosphate. Les sphingolipides sont constitués d'un alcool aliphatique aminé, produit *de novo* à partir de la sérine et d'une acyl-coenzyme A à longue chaîne, et convertie entre autres en céramides, phosphosphingolipides, glycosphingolipides.

### « Glycolipide »

Un glycolipide est un saccharide relié, préférentiellement par un groupement phosphate, à un lipide.

### « Polymérisation in situ »

Par l'expression « polymérisation *in situ* », il est compris selon la présente invention que la polymérisation se fait après injection dans un organisme vivant, tel qu'un être humain.

Ceci est réalisable lorsque les conditions de la polymérisation sont celles du milieu physiologique de l'organisme vivant en question (pH, température, eau, tampon, etc). L'introduction du précurseur non polymérisé dans l'organisme vivant peut s'effectuer simplement en faisant couler ce précurseur sur ou dans l'organisme vivant, si celui-ci a été ouvert au préalable à l'aide d'instruments chirurgicaux. Alternativement, le précurseur de l'hydrogel peut être introduit dans l'organisme vivant en l'injectant à l'aide d'une seringue et d'une aiguille creuse adaptées. Le précurseur de l'hydrogel polymérise alors une fois en contact avec ledit organisme vivant. De manière avantageuse selon la présente invention, l'injection est peu douloureuse, car le précurseur peut être choisi pour être très fluide et donc, le moyen d'injection (seringue et surtout diamètre de l'aiguille) peut être de taille réduite favorisant son introduction dans l'organisme vivant de manière peu douloureuse. En outre, le liquide à injecter étant moins visqueux que si l'hydrogel a déjà été formé au préalable, le liquide une fois injecté prend beaucoup plus facilement sa place, sans distordre les tissus avoisinants, résultant en une injection moins douloureuse.

### Description détaillée

Les caractéristiques physico-chimiques exceptionnelles des hydrogels selon la présente invention permettent des applications pharmaceutiques et biomédicales, notamment dans l'administration de médicaments (dans des nanosphères ou nanocapsules, par voie orale ou voie transdermique...). Les hydrogels selon la présente invention peuvent également être utilisés dans la lutte contre les brûlures cutanées. Ils peuvent aussi être utilisés pour une large gamme d'applications dans les essais cliniques de la médecine expérimentale comprenant : l'ingénierie tissulaire et la médecine de la régénération, le diagnostic, l'immobilisation cellulaire, la séparation de biomolécules ou de cellules, l'utilisation de matériaux "barrière" pour réglementer les adhérences biologiques.

Ainsi l'objet de la présente invention concerne un procédé de fabrication d'hydrogels tel que défini présentement caractérisé en ce que le groupement A de la formule (I) de l'hydrogel tel que défini est choisi parmi les protéines, les peptides tels que des séquences peptidiques issues des collagènes par exemple les séquences comprenant des répétitions de tripeptides Pro-Hyp-Gly ou Pro-Pro-Gly ou Asp-Pro-Gly ou Pro-Lys-Gly, des séquences peptidiques d'autoassemblage comme Arg-Ala-Asp-Ala (Seq ID 4), des oligoprolines, des oligoalanines, des polysaccharides, tels que l'acide hyaluronique* et ses dérivés, des oligonucléotides, des polymères de C₁-C₆-alkylènes-glycols, ou de polyvinylpyrrolidone.

* L'acide hyaluronique peut être utilisé pour ses propriétés structurantes et/ou pour ses activités biologiques.

L'objet de la présente invention concerne en outre un procédé de fabrication d'un hydrogel tel que défini ci-dessus, caractérisé en ce que le groupement B de la formule (II) de l'hydrogel tel que défini présentement est une biomolécule choisie parmi un peptide, un oligopeptide, une protéine, telle que le collagène, un acide désoxyribonucléique, un acide ribonucléique, un polysaccharide, tel qu'une pectine, un chitosane, un acide hyaluronique, un polysaccharide polyarabinose et polygalactose et un glycolipide.

De manière préférée, le fragment B de la formule (II), est un agent antimicrobien, antibiotique et/ou antifongique listés plus haut. De manière plus préférée le fragment B de la formule (II), est plus particulièrement un peptide antimicrobien choisi parmi les amphipathiques, les cationiques, la daptomycine, la polymyxine, la tachyplésine, la magainine, les défensines, les cathélicidines, les histatines, les cécropines, la mellitine, la temporines, les bombinines.

L'objet de la présente invention concerne de plus un procédé de fabrication d'un hydrogel tel que défini ci-dessus, caractérisé en ce que le procédé de polymérisation sol-gel est effectuée à pH physiologique, c'est-à-dire à un pH compris entre pH 6 et 9, préférentiellement entre pH 7 et 8, et encore plus préférentiellement à pH 7,4 ± 0.1, ou en ce que l'hydrogel est formé en présence d'une quantité suffisante d'eau pour que la teneur en eau de l'hydrogel soit d'au moins 50% en poids vis-à-vis du poids total de l'hydrogel formé.

L'objet de la présente invention concerne d'ailleurs un procédé de fabrication d'un hydrogel tel que défini ci-dessus, caractérisé en ce que l'hydrogel est formé en présence d'une quantité suffisante d'eau pour que la teneur en eau de l'hydrogel soit d'au moins 50% en poids vis-à-vis du poids total de l'hydrogel formé. De manière avantageuse, l'hydrogel selon la présente invention peut contenir entre 60 et 99% en poids d'eau vis-à-vis du poids total de l'hydrogel formé selon la présente invention, de manière plus avantageuse l'hydrogel selon la présente invention peut contenir entre 70 et 98% en poids d'eau et de manière encore plus avantageuse l'hydrogel selon la présente invention peut contenir entre 75 et 97%, voire entre 80 et 95% en poids d'eau vis-à-vis du poids total de l'hydrogel formé.

L'objet de la présente invention concerne également un procédé de fabrication d'un hydrogel tel que défini ci-dessus, caractérisé en ce que ledit hydrogel est polymérisé sur ou dans au moins un premier hydrogel en tant que support, résultant ainsi en un hydrogel multicouches. Toute technique connue de l'homme du métier afin d'obtenir un multicouche est applicable en l'espèce. De manière simple, un premier hydrogel peut être coulé dans un fond de moule, puis un second hydrogel coulé sur ce premier hydrogel et ainsi de suite. En outre, les hydrogels selon la présente invention pouvant être découpés et façonnés à volonté, ce découpage peut avoir lieu avant la coulée d'un hydrogel supplémentaire sur un premier hydrogel ou multicouche découpé/façonné.

L'objet de la présente invention concerne en outre un hydrogel pour son utilisation à des fins thérapeutiques et/ou chirurgicales, telle que décrite ci-dessus, caractérisé en ce qu'il permet la délivrance et/ou le transport de molécules actives, ou pour son utilisation *in vivo* en ingénierie tissulaire.

### Figures

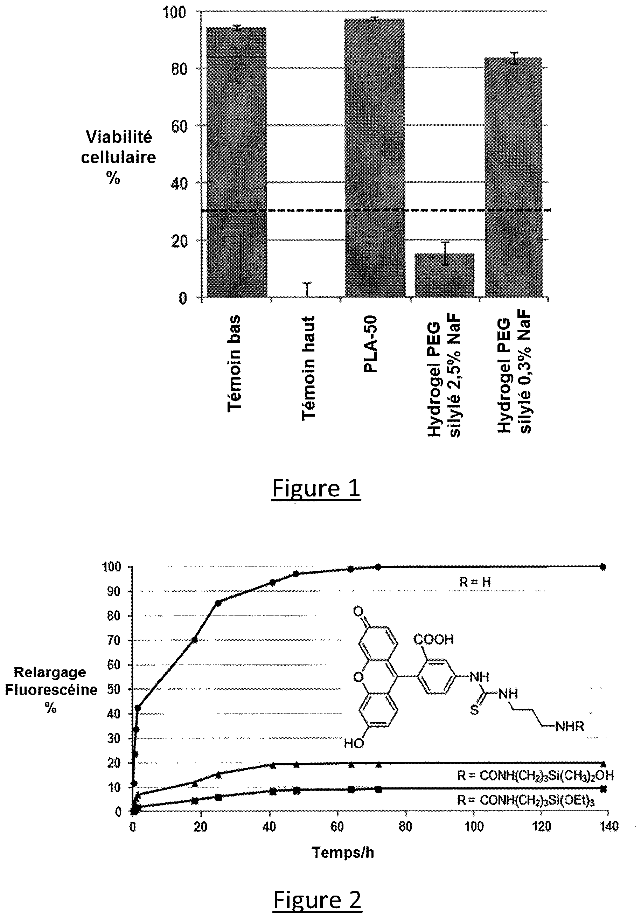

Figure 1 : cette figure concerne des essais de cytotoxicité sur des fibroblastes L929. Le témoin « TC-PS » (colonne « témoins bas » dans la figure) représente la viabilité cellulaire sur TC-PS (i.e. sans hydrogel). Le témoin « cellules lysées» (colonne « témoins haut » dans la figure) correspond à la lyse complète des cellules et donc à un maximum de toxicité. Le témoin « PLA-50 » permet de vérifier la viabilité cellulaire en présence de poly (acide lactique). L'échantillon « hydrogel PEG silylé 2,5% NaF » concerne les cellules incubées 24 h en présence d'un hydrogel contenant 10% en masse de PEG silylé obtenu avec 2,5% en poids de NaF. L'échantillon « hydrogel PEG silylé 0,3% NaF » concerne les cellules incubées 24 h en présence d'un hydrogel contenant 10% en masse de PEG silylé obtenu avec 0,3% en poids de NaF.
Figure 2 : cette figure concerne des essais pour quantifier le relargage de fluorescéine à partir de différents hydrogels selon l'invention. Les hydrogels greffés par de la fluorescéine par liaison covalente affichent des taux de relargage bien moindres que l'hydrogel contenant de la fluorescéine simplement emprisonnée sans liaison covalente.

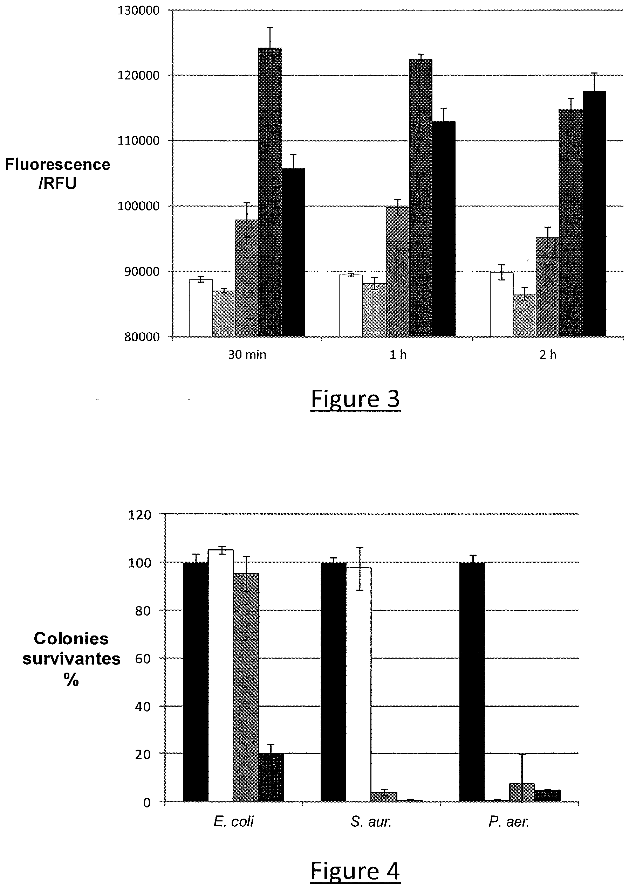

Figure 3 : cette figure concerne des essais d'adhésion cellulaire sur les hydrogels selon la présente invention. Deux contrôles ont été choisis pour comparaison : l'un indique les valeurs mesurées de fluorescence du milieu de culture en l'absence de cellules et l'autre est la fluorescence mesurée pour les cellules déposées directement sur TC-PS. Sur cette base comparative, trois séries d'essais ont été réalisées avec des hydrogels de PEG silylé selon l'invention à 10% en masse de PEG silylé nus, puis avec 7,5% molaire ou 15 % molaire (par rapport au nombre de moles de PEG silylé) d'un peptide permettant l'adhésion cellulaire (« RGD ») lié par liaison covalente audit hydrogel.
   Légende figure 3:
   - Colonnes (de l'histogramme) en blanc (i.e. les 1^{ères} colonnes en partant de la gauche de l'histogramme): milieu de culture en absence de cellules ;
   - Colonnes en gris clair (i.e. les 2^{èmes} colonnes en partant de la gauche de l'histogramme): cellules déposées sur hydrogel de PEG nu ;
   - Colonnes en gris moyen (i.e. les 3^{èmes} colonnes en partant de la gauche de l'histogramme): cellules déposées sur hydrogel de PEG contenant 7.5% de peptide hybride RGD ;
   - Colonnes en gris foncé (i.e. les 4^{èmes} colonnes en partant de la gauche de l'histogramme): cellules déposées sur hydrogel de PEG contenant 15% de peptide hybride RGD ;
   - Colonnes en noir (i.e. les 5^{èmes} colonnes en partant de la gauche de l'histogramme): cellules déposées sur TCPS.
Figure 4 : cette figure concerne des essais antibactériens avec des hydrogels selon la présente invention. Les bactéries ayant fait l'objet du test sont E. coli, S. aureus et P. *aeruginosa.* Deux essais témoins ont été réalisés : l'un étant une évaluation du nombre de colonies bactériennes sur gel d'agar simple, l'autre sur l'hydrogel PEG silylé nu selon la présente invention (i.e. un hydrogel de PEG silylé à 10% en masse de PEG silylé). Un peptide antibactérien a été greffé sur ce même gel (i.e. hydrogel PEG nu) par liaisons covalentes à différents taux (7,5% en moles de peptide antibactérien et 15% en moles de peptide antibactérien par rapport au nombre de moles de PEG silylé). Les colonies bactériennes qui se sont formées au contact des gels sont comptées après 24 h d'incubation à 37°C.
   Légende figure 4:
   - Colonnes (de l'histogramme) en noir (i.e. les 1^{ères} colonnes en partant de la gauche de l'histogramme): colonies ensemencées sur Agar ;
   - Colonnes en blanc (i.e. les 2^{èmes} colonnes en partant de la gauche de l'histogramme): colonies ensemencées sur hydrogel PEG nu ;
   - Colonnes en gris clair (i.e. les 3^{èmes} colonnes en partant de la gauche de l'histogramme): colonies ensemencées sur hydrogel PEG avec 7.5% de peptide hybride antibactérien ;
   - Colonnes en gris foncé (i.e. les 4^{èmes} colonnes en partant de la gauche de l'histogramme): colonies ensemencées sur hydrogel PEG avec 15% de peptide hybride antibactérien.
Figure 5 : illustre une vue Cryo-MEB de l'hydrogel contenant le peptide bi-silylé préparé.
Figure 6 : illustre l'adhésion des cellules mMSC (cellules souches mésenchymateuses murines) sur l'hydrogel selon l'invention, sur des mousses de collagène et du TC-PS. Les histogrammes gris foncé représentent le support TC-PS. Les histogrammes avec des points noirs représentent le support hydrogel selon l'invention. Les histogrammes avec traits obliques noirs représentent le support commercial type collagène.
Figure 7 : illustre en effet la prolifération de mMSC sur l'hydrogel selon l'invention, sur mousse de collagène et TC-PS. La légende concernant les histogrammes est la même que pour la Figure 6.

### Exemples

Les exemples ci-dessous ne limitent en rien la portée de la protection convoitée et sont là à titre d'illustration selon la présente invention.

### Abréviations

ACN, acétonitrile ; Ahx, acide ε-aminohexanoïque ; Boc, t-Butyloxycarbonyl ; DCM, dichlorométhane ; DIEA, diisopropyléthylamine ; DMF, N-N'-diméthylformamide ; DPBS, Dulbelcco's phosphate buffered saline ; ESI-MS, spectrométrie de masse avec ionisation électrospray ; Fmoc, fluorénylméthoxycarbonyl ; HBTU, *N*,*N*,*N'*,*N'*-tétraméthyl-*O*-(1*H-*benzotriazol-1-yl)uronium hexafluorophosphate ; HPLC, chromatographie liquide haute performance ; HRMS, spectrométrie de masse haute résolution ; LC/MS, spectrométrie de masse couplée à la chromatographie liquide ; Pbf, 2,2,4,6,7-pentaméthyldihydrobenzofuran-5-sulfonyl ; PEG, polyéthylène glycol ; pip, pipéridine; PS, polystyrène; RMN, résonnance magnétique nucléaire ; TA, température ambiante , c'est-à-dire compris entre 20 et 25°C; TFA, acide trifluoroacétique; THF, tétrahydrofurane; TIS, triisopropylsilane.

### Matériel et méthodes

Les HPLC analytiques ont été réalisées sur un appareil Agilent Infinity 1260 équipé d'une barette de diode et d'une colonne Kinetex C18 phase inverse, 2,6 µm, 50 x 4.6 mm avec un gradient de 0% à 90% (% en volume) de B en 5 min avec éluant A : eau/0.1% TFA et éluant B : ACN/0.1% TFA à un débit de 2,5 mL/min.

Les purifications par HPLC préparative ont été réalisées sur un appareil Waters HPLC 4000, équipé d'un détecteur UV 486 et d'une colonne en phase inverse C₁₈ Waters Delta-Pack 40×100 mm, 100 Å, 15 µm, à un débit de 50 mL/min. Les solvants utilisés sont H₂O/0.1% TFA et ACN/0.1% TFA.

Les échantillons pour les analyses LC/MS ont été préparés dans un mélange eau/ACN (50:50, v/v) contenant 0.1% TFA. L'appareillage LC/MS est constitué d'une HPLC Waters Alliance 2695, couplée à un spectromètre Water Micromass ZQ (ionisation électrospray, mode positif). Les analyses ont été réalisées avec une colonne phase inverse Phenomenex Onyx, 25 x 4.6 mm à un débit de 3 mL/min avec un gradient de 0 à 100% (% en volume) de B en 2,5 min avec éluant A : eau/0.1% HCO₂H ; éluant B : ACN/0.1% HCO₂H. La détection UV a été faite à 214 nm. Les spectres de masse ont été acquis avec un débit de solvant de 200 µL/min. L'azote est utilisé comme gaz nébulisant et séchant. Les données sont obtenues en scannant les m/z de 100 à 1000 en 0,75 secondes ou de 200 à 1600 en 0,9 secondes. Les analyses de spectrométrie de masse haute résolution ont été réalisées en mode positif sur un spectromètre temps de vol (TOF) équipé d'une source d'ionisation électrospray.

Les spectres RMN ¹H, ¹³C et ²⁹Si ont été enregistrés à température ambiante (TA) dans des solvants deutérés sur un spectromètre à 400, 101 et 79 MHz respectivement. Les déplacements chimiques (δ) sont donnés en parties par million en utilisant les solvants non deutérés résiduels comme références (CHCl₃ dans CDCl₃, δH = 7.26 ppm; DMSO-d6, δH = 2.50 ppm). Les signaux sont notés s (singulet), d (doublet), t (triplet), q (quadruplet), dt (triplet dédoublé), m (multiplet)... Les constantes de couplage sont mesurées en Hertz.

### Accrochage d'un acide aminé sur la résine 2-chlorochlorotrityle :

La résine 2-chlorochlorotrityle (1,44 mmol Cl/g, 1 éq) est placée dans un réacteur de synthèse peptidique en phase solide équipé d'un fritté. L'acide aminé protégé Fmoc-AA-OH (3 éq) est couplé sur la résine en présence de DIEA (5 éq) dans le DMF pendant une nuit. Après les lavages standards (3×DMF, 1×MeOH et 1×DCM), la résine Fmoc-AA-Cltrityle est séchée sous vide pendant 12 h. La charge de la résine est déterminée par détection à 299 nm de l'adduit pipéridine-dibenzofulvène qui se forme dans la solution de déprotection pip/DMF (20/80 v/v).

### Déprotection du Fmoc :

La résine Fmoc-Rink Amide AM PS ou Fmoc-peptidyl-résine est place dans un réacteur de phase solide muni d'un fritté. Le groupement Fmoc est retiré par deux traitements successifs avec une solution de DMF-pipéridine (80:20; v/v, 2 x 20 min). Entre les deux traitements, la solution est filtrée et remplacée par une solution fraîche. Les étapes classiques de lavage sont réalisées en fin de déprotection (3×DMF, 1×MeOH et 1×DCM).

### Couplage d'un amino acide :

L'acide aminé protégé en N-ter par un groupement Fmoc (3 éq) est dissous dans le DMF (10 mL par g de résine) en présence d'HBTU (3 éq) et de DIEA (3 éq) pendant 10 min. Cette solution est ajoutée sur la peptidyl-résine dont le N-ter est libre. La résine est agitée à TA pendant 1h30 puis lavée (3×DMF, 1×MeOH et 1×DCM).

### Exemple 1 : Silylation de PEG

Du polyéthylène glycol de masse moléculaire moyenne 2000 g/mol (2,00 g, 1,00 mmol) est séché sous vide à 80°C pendant une nuit puis il est dissous dans du THF anhydre (12 mL) sous argon. De la triéthylamine (1,66 mL, 12 mmol, 12 éq) et l'isocyanatopropyltriéthoxysilane (744 µL, 3 mmol, 3 éq) sont ajoutés. Le mélange est chauffé à reflux pendant 48 h puis concentré sous pression réduite. Le PEG bi-silylé est alors précipité dans l'hexane. Après centrifugation, il est lavé 3 fois à l'hexane et séché sous vide. Il est obtenu sous forme d'une poudre blanche stockée à 4°C sous argon. ¹H RMN (400 MHz, CDCl₃) δ 5.00 (sl, 2H, NH), 4.18 (t, J = 4.7 Hz, 4H, H-6), 3.79 (q, J = 7.0 Hz, 12H, H-2), 3.62 (s, 177H, CH₂ PEG), 3.14 (dd, J = 13.2, 6.7 Hz, 4H, H-5), 1.58 (qu, J = 7.7 Hz, 4H, H-4), 1.20 (t, J = 7.0 Hz, 18H, H-1), 0.64 - 0.54 (m, 4H, H-3).¹³C RMN (101 MHz, CDCl₃) δ 156.72 (C), 70.55 (CH₂), 63.76 (CH₂), 58.26 (CH₂), 43.29 (CH₂), 23.12 (CH₂), 18.38 (CH₃), 9.12 (CH₂). ²⁹Si RMN (79 MHz, CDCl₃) δ -45.73 (s).

### Exemple 2 : Synthèse d'un peptide dérivé du collagène et bi-silylé

### Préparation du tripeptide : Fmoc-Pro-Hyp-Gly-OBzl

### Couplage de Boc-Hyp-OH :

Dans un ballon monocol de 5000 mL sont introduits H-Gly-OBzl.HCl (11,42 g, 56,6 mmol, 1éq) dissous dans AcN et de la DIEA (37,44 mL, 226,5 mmol, 4 éq). Dans un bécher, Boc-Hyp-OH (13,3 g, 56,6 mmol) est dissous dans AcN. La DIEA ainsi que le pyBOP (29,3 g, 56,63 mmol, 1 éq) sont ajoutés à cette solution. Les deux acides aminés sont mis en contact et agités à TA pendant 4h. Le suivi de la réaction est effectué par HPLC analytique.

Une fois la réaction terminée, AcN est évaporé et l'huile orangée obtenue est solubilisée dans de l'acétate d'éthyle. Cette solution est lavée par des solutions aqueuses de KHSO₄, NaHCO₃ et NaCl. La phase organique est ensuite séchée avec MgSO₄ et le solvant évaporé sous pression réduite.

### Déprotection N-ter du dipeptide :

Le produit est mis ensuite en solution dans 150 ml de TFA pendant 40 min jusqu'à disparition complète de dégagement gazeux. Le TFA est alors évaporé sous pression réduite et le dipeptide est précipité dans l'éther diéthylique puis lyophilisé.

### Couplage de Fmoc-Pro-OH :

Fmoc-Pro-OH est couplé sur H-Hyp-Gly-OBzl selon le même protocole que le couplage de Boc-Hyp-OH décrit ci-dessus. La réaction dure 2 h. A l'issue des lavages, le tripeptide est purifié sur gel de silice (appareil Biotage, colonne SNAP 340 g, gradient de 0% de MeOH dans le DCM à 10% de MeOH dans le DCM, sortie du produit à 50 % du gradient, rendement 71%).

### Déprotection C-ter :

Dans un ballon monocol de 250 mL sont introduits Fmoc-Pro-Hyp-Gly-OBzl (20,4 g, 40,2 mmol) dissous dans EtOH et 200 mg de Pd/C. Le tout est mis sous bullage d'hydrogène pendant 6h à 60°C. Le suivi de la réaction est effectué par HPLC analytique. Une fois la réaction terminée, la solution est filtrée sur célite puis concentrée sous pression réduite. Le solide est repris dans H₂O/AcN 50/50 V/V et lyophilisé (Rendement : 86%).

### Synthèse du peptide Ac-Lys-(Pro-Hyp-Gly)₃-Lys-NH₂ (Seq ID 5) sur support à partir du bloc tripeptidique

La synthèse s'effectue dans une seringue munie d'un fritté sur une résine Rink Amide PS dont la charge est de 0,94 mmol/g avec une échelle de synthèse de 1 mmol. La résine est gonflée dans le DCM puis lavée au DMF. Elle est déprotégée grâce à deux traitements avec la solution de déprotection pip/DMF (15 mL pendant 5 min, 3 lavages au DMF, 15 mL pendant 20 min puis lavages (3×DMF, 3×DCM, 1×DMF)). Les conditions de couplage habituelles sont légèrement modifiées. Les couplages sont allongés à 2 h et se font avec 1,5 éq de Fmoc-Lys(Boc)-OH ou Fmoc-(Pro-Hyp-Gly)₃-OH, 5 éq de DIEA et 1,5 éq d'HATU qui remplace l'HBTU. En fin de couplage, la résine est lavée avec les solvants suivants : (3xDMF, 3xDCM, 1×DMF). Le dernier couplage est lui aussi suivi d'une déprotection du groupement Fmoc. Puis la peptidyl-résine est acétylée avec de l'acide acétique (2 éq) dans le DCM (10 mL) en présence de BOP (2 éq) et de DIEA (4 éq) pendant 1h30. La résine est lavée puis clivée dans un mélange TFA/TIS/H₂O (95/2,5/2,5 v/v/v, 50 ml). La solution de "clivage" est concentrée sous pression réduite et le peptide est précipité à l'éther. Après centrifugation et élimination du surnageant, le peptide brut est repris dans un mélange eau/ACN et lyophilisé. Il est enfin purifié par HPLC préparative sur une colonne Luna C₁₈ phase inverse (15 µm, 250 x 50 mm) à un débit de 120 mL/min avec un gradient de 0 à 6% de B en 6 min, de 6% à 10% de B en 8 min et de 10% à 18% en 24 min avec éluant A : H₂O/0,1% TFA et éluant B : ACN/0,1% TFA. Rendement : 49% ; pureté > 99%. LC/MS (ESI⁺): t_{R} = 0.73 min, 1118 ([M+H]⁺, 5%), 559 ([M+2H]²⁺, 100).

### Silylation

Ac-Lys-(Pro-Hyp-Gly)₃-Lys-NH₂ (20 mg, 14,9 µmol) est dissous dans le diméthylformamide anhydre (300 µL) sous argon. La diisopropyléthylamine (12,4 µL, 71,3 µmol, 4,8 éq) puis le 3-isocyanatopropyltriéthoxysilane (9,7 µL, 39,3 µmol, 2,6 éq) sont ajoutés à la solution de nonapeptide. Le mélange réactionnel est laissé 50 min sous agitation. La fin de la réaction est contrôlée par LC/MS. Le solvant est évaporé sous pression réduite. Puis le nonapeptide silylé est précipité à l'éther diéthylique. Après centrifugation, le peptide hybride est lavé à 3 reprises à l'éther diéthylique puis la poudre obtenue est séchée sous vide. LC/MS (ESI⁺): t_{R} = 0.82 min, 704 ([M+2H-2H₂0]²⁺, 100%), 695 ([M+2H-3H₂0]]²⁺, 80) et t_{R} = 0.87 min (conformère), 704 ([M+2H-2H₂0]²⁺, 70%), 695 ([M+2H-3H₂0]]²⁺, 100).

### Exemple 3 : Préparation d'un hydrogel constitué d'une molécule de formule (I)

Une molécule de formule (I), par exemple le PEG bi-silylé préparé dans l'exemple 1 ou le peptide bi-silylé dérivé du collagène synthétisé dans l'exemple 2, est dissous dans du tampon phosphate à pH 7,4 (DPBS Dulbecco's phosphate buffered saline), de préférence à une concentration de 10 % en masse, en présence de fluorure de sodium (3 mg de NaF par mL de DPBS). La solution non visqueuse est incubée à 37°C, il se forme alors un gel. Le temps de gélification dépend de la nature de la molécule de formule (I) et de sa concentration.

### Exemple 4 : Synthèse du peptide silylé (EtO)₃Si-(CH₂)₃-NHCO-(βAla)₄-Gly-Arg-Gly-Asp-Ser-Pro-OH (Seq ID 6)

H-(βAla)₄-Gly-Arg-Gly-Asp-Ser-Pro-OH (Seq ID 6) est synthétisé sur une résine 2-chlorochlorotrityle (charge: 1,44 mmol/g, échelle de synthèse: 0,5 mmol) en stratégie Fmoc/tBu. Les acides aminés utilisés sont successivement Fmoc-Pro-OH, Fmoc-Ser(tBu)-OH, Fmoc-Asp-(tBu)-OH, Fmoc-Gly-OH, Fmoc-Arg(Pbf)-OH, Fmoc-Gly-OH et Fmoc-βAla-OH quatre fois. Chaque couplage d'acide aminé est suivi d'une déprotection du groupement Fmoc en N-ter. Le "clivage" du peptide et la déprotection des chaînes latérales sont effectués dans un mélange TFA/TIS/H₂O 95/2.5/2.5 V/V/V pendant 4 h. Après précipitation, le peptide est purifié par HPLC préparative sur une colonne C₁₈. Il est obtenu après lyophilisation avec un rendement de 63%. Il est ensuite silylé par réaction avec le 3-isocyanatopropyltriéthoxysilane (1,1 éq) dans le DMF à une concentration de 30 mM en présence de DIEA (3 éq). La réaction est suivie par HPLC analytique. Le DMF est ensuite évaporé sous pression réduite et le peptide silylé est précipité dans l'éther diéthylique. Il est récupéré par centrifugation à l'issue de 3 lavages dans l'éther diéthylique.
¹H RMN (400 MHz, DMSO-d6) δ 8.61 - 8.40 (m, 2H, NH Asp et Gly), 8.21 - 8.04 (m, 2H, NH Gly N-ter et Arg), 7.99 - 7.78 (m, 3H, NH βAla)), 7.58 - 7.42 (m, 1H, NH Ser), 7.34 - 6.98(m, 3H, OH Ser, COOH Asp et C-ter), 5.97 (t, J = 5.6 Hz, 1H, NH urée), 5.78 (t, J = 5.6 Hz, 1H, NH urée βAla), 4.56 (q, J = 6.9 Hz, 1H, Hα Ser), 4.49 - 4.38 (m, 1H, Hα Asp), 4.38 - 4.26 (m, 1H, Hα Arg), 4.22 (dd, J = 8.7, 4.1 Hz, 1H, Hα Pro), 4.02 - 3.75 (m, 4H, Hα Gly), 3.73 (q, J = 6.9 Hz, 6H, CH₂ éthoxy), 3.67 - 3.46 (m, 4H, Hδ Pro et HB Ser), 3.46 - 3.28 (m, 2H, Hδ Arg), 3.28 - 3.11 (m, 8H, HB βAla), 2.92 (q, J = 6.1 Hz, 2H, H-3), 2.60 - 2.48 (m, 2H, HB Asp), 2.29 (t, J = 7.1 Hz, 2H, Hα βAla), 2.26 - 2.09 (m, 6H, Hα βAla), 1.87 (dd, J = 13.3, 6.5 Hz, 2H, Hγ Pro), 1.60 - 1.56 (m, 2H, Hβ Arg), 1.55 - 1.42 (m, 2H, Hδ Arg), 1.38 (m, 2H, H-2), 1.19 - 1.09 (t, J = 7.2 Hz, 9H, CH₃ éthoxy), 0.55 - 0.42 (m, 2H, H-1). ¹³C RMN (101 MHz, DMSO-d6) δ 174.27 (C), 172.82 (C), 171.38 (C), 171.27 (C), 171.14 (C), 170.86 (C), 170.80 (C), 169.58 (C), 169.43 (C), 169.02 (C), 168.77 (C), 158.41 (C), 157.52 (C), 62.14 (CH₂), 59.54 (CH), 58.14 (CH₂), 53.51 (CH), 52.60 (CH), 49.78 (CH), 47.02 (CH₂), 42.46 (CH₂), 42.36 (CH₂), 40.89 (CH₂), 36.68 (CH₂), 36.30 (CH₂), 35.88 (CH₂), 35.76 (CH₂), 30.10 (CH₂), 29.13 (CH₂), 25.39 (CH₂), 24.91 (CH₂), 24.01 (CH₂), 18.68 (CH₃), 7.72 (CH₂). ²⁹Si RMN (79 MHz, DMSO-d6) δ -45.10. LC/MS (ESI⁺): Seuls les produits d'hydrolyse des groupements éthoxysilanes en silanols sont détectés. t_{R} = 0.62 min, 1035 ([M+H]⁺, 50%), 509 ([M+H-OH]²⁺, 60), 500 ([M-2OH]²⁺, 100). HRMS: 1119.5481. C₄₅H₇₄N₁₈O₁₄Si implique [M+H]⁺, 1119.5479.

### Exemple 5 : Synthèse du peptide silylé HO(CH₃)₂Si-(CH₂)₃-NHCO-Ahx-Arg-Arg-NH₂

Le peptide antibactérien H-Ahx-Arg-Arg-NH₂ est synthétisé sur une résine Rink Amide (charge 0,94 mmol/g, échelle de synthèse : 3 mmol) en stratégie Fmoc/tBu. Les acides aminés utilisés sont successivement Fmoc-Arg(Pbf)-OH deux fois et l'acide Fmoc-ε-aminohexanoïque. Chaque couplage est suivi d'une déprotection du groupement Fmoc en N-ter. Le tripeptide est ensuite silylé sur support dans le DMF (10 mL/g de résine) en utilisant le 3-isocyanatopropyldiméthylchlorosilane (3 éq) en présence de DIEA (3 éq). La réaction de silylation est laissée sous agitation pendant une nuit puis la résine est lavée (3×DMF, 1×MeOH et 1×DCM) et clivée dans le TFA pendant 5 h. La solution de « clivage » est concentrée sous pression réduite puis le peptide antibactérien silylé est précipité dans l'éther diéthylique et enfin purifié par HPLC préparative sur une colonne C₁₈ (Eluant A : H₂O/0,1% TFA, éluant B : ACN/0,1% TFA, gradient : de 0 à 7% de B en 2 min puis de 7% à 30% de B en 23 min, sortie du produit à 16% de B). Après lyophylisation, le peptide antibactérien silylé est obtenu sous forme d'une poudre blanche (Rendement : 68%, pureté > 98%). ¹H RMN (400 MHz, D₂O) δ 4.19 (ddd, *J* = 14.4, 8.6, 5.7 Hz, 2H, Hα Arg), 3.09 (t, *J* = 6.9 Hz, 4H, Hδ Arg), 2.96 (td, *J* = 6.8, 1.9 Hz, 4H, H-3 et H-4), 2.17 (t, *J* = 7.3 Hz, 2H, H-8), 1.82 - 1.59 (m, 4H, Hβ Arg), 1.59 - 1.44 (m, 6H, H-7 et Hγ Arg), 1.41 - 1.31 (m, 4H, H-2 et H-5), 1.24 - 1.12 (m, 2H, H-6), 0.53 - 0.41 (m, 2H, H-1), 0.00 (s, 6H, Si(CH₃)₂). ¹³C RMN (101 MHz, DMSO-d6) δ 172.40 (C), 171.76 (C), 170.69 (C), 157.38 (C), 156.00 (C), 51.38 (CH), 51.02 (CH), 41.54 (CH₂), 34.28 (CH₂), 29.01 (CH₂), 28.34 (CH₂), 28.08 (CH₂), 25.24 (CH₂), 24.24 (CH₂), 23.16 (CH₂), 14.24 (CH₂), -0.51 (CH₃). ²⁹Si RMN (79 MHz, DMSO-d6) δ 7.97 (dimère). LC/MS (ESI⁺): t_{R} = 0.71 min, 602 ([M+H]⁺, 10%), 302 ([M+2H]²⁺, 100), 293 ([M+2H-NH₃]²⁺, 30). HRMS: 602.3926. C₂₄H₅₁N₁₁O₅Si implique [M+H]⁺, 602.3922.

### Exemple 6 : Synthèse d'une hydroxydiméthylsilyl fluorescéine

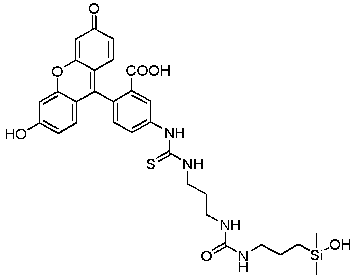

La N-Boc-1,3-propanediamine (56,4 mg, 0,324 mmol, 1,05 éq) est ajoutée à une solution de fluorescéine isothiocyanate (FITC, 120 mg, 0,308 mmol) dans le DMF anhydre (3 mL) en présence de DIEA (100 µL). Le mélange réactionnel est agité pendant 1 h à TA sous argon. Puis le DMF est évaporé sous pression réduite. La Boc-amino fluorescéine est précipitée et lavée dans l'éther diéthylique puis séchée. Elle est ensuite solubilisée dans le TFA (4 mL) et cette solution est agitée pendant 1 h. Le mélange réactionnel est concentré et précipité dans l'éther diéthylique. Après centrifugation, le surnageant est retiré. La fluorescéine amine est purifiée par HPLC préparative sur une colonne C₁₈ (Eluant A : H₂O/0,1% TFA, éluant B : ACN/0,1% TFA, gradient : de 0 à 15% de l'éluant B en 3 min puis de 15% à 40% de l'éluant B en 25 min, sortie du produit à 22% de l'éluant B) et obtenue sous forme de sel de TFA (180 mg, 100%, pureté = 95%).

Le 3-isocyanatopropylchlorodiméthylsilane (16,2 µL, 0,0910 mmol, 1,05 éq) est ajouté à une solution de fluorescéine amine purifiée précédemment (50,0 mg, 0,0867 mmol) dans le DMF anhydre (2 mL) en présence de DIEA (45,2 µL, 0,260 mmol, 3 éq). Le mélange réactionnel est agité sous argon pendant 1 h. Le solvant est évaporé sous pression réduite et le produit brut est obtenu par précipitation dans l'éther diéthylique. La hydroxydiméthylsilyl fluorescéine est purifiée par HPLC préparative sur une colonne C₁₈ (Eluant A : H₂O/0,1% TFA, éluant B : ACN/0,1% TFA, gradient : de 0 à 20% de l'éluant B en 4 min, de 20 à 26% de l'éluant B en 6 min puis de 26% à 46% de l'éluant B en 30 min, sortie du produit à 32% de l'éluant B, rendement : 42%, pureté : 97%) ¹H RMN (400 MHz, DMSO-d6) δ 10.01 (sl, 1H, COOH), 9.97 (s, 1H, NH thiourée provenant de la FITC), 8.20 (s, 1H, H-4), 8.12 (s, 1H, NH thiourée), 7.71 (d, *J* = 7.6 Hz, 1H, H-6), 7.14 (d, *J* = 8.3 Hz, 1H, H-5), 6.65 (d, *J* = 2.2 Hz, 2H, H-9 et H-10), 6.59 - 6.51 (m, 4H, H-7, H-8, H-11 et H-12), 5.88 (s br, 2H, NH urée), 3.43 - 3.38 (m, 2H, H-3), 3.03 (t, *J* = 6.5 Hz, 2H, H-1), 2.92 (t, *J* = 6.9 Hz, 2H, H-3'), 1.62 (qu, *J* = 6.5 Hz, 2H, H-2), 1.37 - 1.29 (m, 2H, H-2'), 0.47 - 0.36 (m, 2H, H-1'), 0.00 et -0.04 (2 s, 6H, H-4' du dimère et du monomère respectivement). ¹³C RMN (101 MHz, DMSO-d6) δ 179.51 (C), 167.65 (C), 158.62 (C), 157.51 (C), 151.02 (C), 140.47 (C), 128.75 (CH), 128.19 (CH), 125.68 (C), 123.18 (CH), 116.66 (C), 115.79 (CH), 114.24 (C), 111.71 (CH), 108.87 (C), 101.36 (CH), 41.55 (CH₂), 40.52 (CH₂), 35.85 (CH₂), 28.80 (CH₂), 23.13 (CH₂), 14.20 (CH₂), -0.51 (CH₃), -0.75 (CH₃). ²⁹Si RMN (79 MHz, DMSO-d6) δ 11.24 (monomère), 7.99 (dimère). LC/MS (ESI⁺): t_{R} = 1.33 min, 623 ([M+H]⁺, 60%), 390 ([M+H-NH₂(CH₂)₃NH-CONH(CH₂)₃Si(CH₃)₂OH]⁺, 15), 312 ([M+2H]²⁺, 60), 303 ([M+2H-H₂O]²⁺, 100. HRMS: 623.1996. C₃₀H₃₄N₄O₇SSi implique [M+H]⁺, 623,1996.

### Exemple 7 : Préparation d'hydrogels comportant une molécule de formule (I) et une molécule de formule (II)

Une molécule de formule (I), par exemple le PEG bi-silylé préparé dans l'exemple 1 ou le peptide bi-silylé dérivé du collagène synthétisé dans l'exemple 2, est dissous dans du tampon phosphate à pH 7,4 (DPBS Dulbecco's phosphate buffered saline), de préférence à une concentration de 10 % en masse, en présence de fluorure de sodium (3 mg de NaF par mL de DPBS). Une molécule de formule (II), par exemple le peptide silylé contenant la séquence Arg-Gly-Asp synthétisé dans l'exemple 4 ou le peptide silylé antibactérien préparé dans l'exemple 5 ou la fluorescéine silylée décrite dans l'exemple 6, est ajouté à la solution de molécule de formule (I) à une concentration comprise entre 1% et 15 mol% par rapport à la molécule de formule (I). La solution non visqueuse est incubée à 37°C, il se forme alors un gel. Le temps de gélification dépend de la nature des molécules choisies et de leur concentration.

### Exemple 8 : exemples d'applications d'un hydrogel selon l'invention

### • Optimisation de la synthèse

L'unité bi-fonctionnelle (EtO)₃-Si-(CH₂)₃-NHCO-(PEG2000)-OCONH-(CH₂)₃-Si-(OEt)₃ (i.e. « bloc PEG hybride bi-silylé ») a été synthétisée en faisant réagir le polyéthylène glycol (PM = 2000 Da) avec du 3-isocyanatopropyltriéthoxysilane. Ensuite, le bloc PEG hybride bi-silylé a été engagé dans le procédé sol-gel, consistant en l'hydrolyse des éthoxysilyles en silanols et leur condensation pour former des liaisons siloxanes. Ce processus a été réalisé à 37 °C, à pH 7,2-7,4 dans un tampon phosphate (DPBS). Le fluorure de sodium (NaF) a été utilisé en tant que catalyseur nucléophile pour accélérer les réactions de condensation. Différentes concentrations de PEG hybride bi-silylé et de fluorure de sodium ont été testées démontrant ainsi leur influence sur le temps de gélification (tableau 1) :

**Tableau 1 : Temps de gélification de solutions de PEG hybride bi-silylé dans le DPBS à 37°C et modules visco-élastiques des hydrogels obtenus**

| Composition du gel | | | | |
|---|---|---|---|---|
| Bloc PEG hybride bi-silylé (% massique) | NaF (% massique) | Temps de gélification (min) | G' (Pa) | G" (Pa) |
| 20 | 5.0 | 10 | n. d. | n. d. |
| 20 | 2.5 | 15 | 77 750 | 204 |
| 10 | 5.0 | 20 | n. d. | n. d. |
| 10 | 2.5 | 35 | 18 960 | 49 |
| 10 | 0.3 | 120 | 9 947 | 61 |
| 5 | 5.0 | 50 | n. d. | n. d. |
| 5 | 2.5 | 220 | 5413 | 39 |

L'effet du rapport PEG/eau et de la concentration en NaF sur les propriétés mécaniques des hydrogels a également été étudié. La réponse viscoélastique des hydrogels a été mesurée en oscillation sur un rhéomètre AR 2000 (TA Instruments, Inc.) avec une géométrie parallèle de 20 mm de diamètre (force normale = 2 N). L'évolution des modules de conservation (G') et de perte (G") a été suivie en fonction de la fréquence d'oscillation dans le domaine linéaire visco-élastique (0,1 % de déformation, de 0,01 Hz à 10 Hz) pour des gels de compositions différentes. Les valeurs des modules pour une fréquence de 10 Hz sont présentées dans le tableau 1. Tous les échantillons présentent les propriétés d'un solide avec G' supérieur à G". Les modules de conservation ont été utilisés comme mesure de l'élasticité des hydrogels. Ils sont restés stables pendant une semaine. S'étendant de 5 000 à 80 000 Pa selon la composition du gel, ils couvrent une large gamme de rigidité. Très peu de variations ont été observées sur les modules de perte pour l'ensemble des échantillons. Ainsi selon l'application visée, la rigidité des gels peut être ajustée en faisant varier les concentrations en PEG hybride bi-silylé et/ou en NaF.

### • Tests de cytotoxicité de l'hydrogel nu

Des essais de cytotoxicité ont été effectués sur deux des hydrogels présentés ci-dessus contenant chacun 10% massique de PEG hybride bi-silylé par rapport à la masse de solvant et 0,3% ou 2,5% massique de NaF respectivement.

Des fibroblastes murins de la lignée L929 ont été ensemencés dans des puits en polystyrène traités pour la culture de tissus. Après 24 h de prolifération, ces cellules ont été incubées avec les hydrogels pendant 24 h supplémentaires. La cytotoxicité a ensuite été mesurée à l'aide d'un test mettant en évidence la libération de lactate déhydrogénase par les cellules. Comme prévu, les hydrogels contenant la plus forte concentration de NaF se sont révélés toxiques. Cependant, plus de 80% de la viabilité cellulaire a été observée pour une concentration en NaF de 3 mg / ml, ce qui signifie que ce dernier hydrogel n'est pas toxique pour les cellules (Figure 1).

Ces résultats ont été confirmés par des observations microscopiques qui ont montré des cellules saines fusiformes.

### • Vérification de la fonctionnalisation de l'hydrogel

Afin de montrer l'incorporation covalente d'une (bio-)molécule dans le gel et l'absence de relargage dans le temps des molécules greffées, des dérivés de fluorescéine ont été choisis pour être liés chimiquement aux hydrogels conformément à la présente invention (cf. figure 2 pour les formules exactes). Ainsi, s'il devait y avoir un relargage des molécules greffées, l'utilisation de la fluorescéine permettrait de le détecter aisément. L'isothiocyanate de fluorescéine (FITC) a été utilisé pour préparer deux types de dérivés de fluorescéine donnant des liaisons covalentes (triéthoxysilane et diméthylhydroxysilane), ainsi qu'une molécule non-silylée servant de témoin/contrôle. Chaque dérivé de la fluorescéine a été dissous à une concentration de 5,2 mM dans une solution de PEG hybride bi-silylé lui-même à une concentration de 10% massique par rapport à la masse de DPBS servant de solvant. Le fluorure de sodium a été ajouté et les solutions ont été homogénéisées. Des hydrogels hybrides fluorescents ont été obtenus à 37 °C en 30 minutes. Les différents hydrogels ont été placés dans du tampon phosphate (10 ml) et la libération de fluorescéine a été contrôlée par HPLC. Dans le cas de l'emprisonnement non-covalent, un relargage complet de fluorescéine a été observé dans les 72h. Comme on s'y attendait, le relargage de fluorescéine est limité (au regard du contrôle) dans le cas des dérivés covalents et atteint un plateau après 72 heures. Ceci indique la stabilité des liaisons covalentes hybrides. Un maximum de 9% et 20% (par rapport à la quantité totale de fluorescéine introduite) de libération a été observé pour les hydrogels obtenus avec la « triéthoxysilylfluorescéine » et la « diméthylhydroxyfluorescéine », respectivement (figure 2). Ce relargage pourrait être attribué à des molécules hybrides piégées de façon non-covalente qui n'auraient pu réagir pendant le processus de gélification. Ceci est cohérent avec le fait que le dérivé triéthoxysilyle devrait être plus réactif que le diméthylhydroxysilyle lors de la réaction de condensation.

### • Test d'adhésion cellulaire

Un peptide contenant la séquence RGD a été choisi pour favoriser l'adhérence cellulaire. Il s'agit de la séquence GRGDSP (SEQ ID 7).

Le peptide déprotégé H-GRGDSP-OH (Seq ID 7) a tout d'abord été préparé par synthèse peptidique sur support solide en stratégie Fmoc / tBu et fonctionnalisé par un groupement triéthoxysilyle en utilisant l'ICPTES (3-isocyanatopropyltriéthoxysilane). Une solution de PEG hybride bi-silylé à 10% massique par rapport à la masse de DPBS) contenant 0,3% massique en NaF a été préparée et le peptide hybride GRGDSP silylé a été ajouté à cette solution. La concentration relative en GRGDSP silylé dans le mélange avant réaction a été fixée à 7,5% (1^{ère} solution) et à 15% (2^{ème} solution) en moles par rapport au nombre de moles de PEG hybride bi-silylé. Ces deux solutions ont été placées à 37 ° C pendant une nuit, pour fournir deux hydrogels de « PEG / RGD-silylés ».

Des fibroblastes L929 ont été ensemencés à la surface des hydrogels de PEG/RGD-silylés et sur un hydrogel hybride de PEG non fonctionnalisé (« hydrogel nu »). Les cellules adhérentes après 30 min, 1 h et 2 h d'incubation ont été mises en évidence et dosées à l'aide d'un réactif de viabilité « PrestoBlue Cell Viability Reagent ® » (figure 3). Aucune adhésion cellulaire n'a été constatée sur l'hydrogel nu. En revanche, l'adhésion cellulaire a été très efficace dans le cas de l'hydrogel contenant 15% molaire en RGD silylé (solution 2). En effet, l'adhésion sur ce dernier après 30 min d'incubation est meilleure que sur le polystyrène traité pour la culture de tissus (TC-PS), et ce pour des surfaces d'adhésion de taille identique.

### • Tests antibactériens

De même, des hydrogels avec des propriétés antibactériennes ont été préparés en utilisant la séquence peptidique H-Ahx-Arg-Arg-NH₂ convenablement silylé du côté N-terminal. Pour ce faire, le peptide H-Ahx-Arg(Pbf)-Arg(Pbf)-NH- sur résine rinkamide a été fonctionnalisé à l'extrémité N-terminale par un groupement diméthylhydroxysilyle avant clivage de la résine et déprotection des chaînes latérales. Le peptide hybride résultant a été ajouté à des solutions de PEG hybride bi-silylé selon le protocole décrit précédemment pour l'hydrogel «PEG-RGD silylés». L'activité antibactérienne des hydrogels a été évaluée contre Escherichia coli, Staphylococcus aureus et Pseudomonas aeruginosa. Les hydrogels ont été inoculés en surface avec des bactéries et recouverts de gélose trypticase soja. Après 24 h d'incubation à 37 ° C, les colonies bactériennes ont été comptées (Figure 4). Les hydrogels de PEG « nus » semblent inhiber la croissance de P. aeruginosa même en l'absence de peptide. Pour E. coli et S. aureus, l'effet antibactérien a été apporté par le peptide greffé. En effet, 15 mol% en peptide antibactérien ont induit une inhibition complète de la croissance de S. aureus et ont réduit la croissance d'E. Coli de 80%.

### • Test d'adhésion et de prolifération cellulaire

Un hydrogel tel qu'obtenu à l'Exemple 3 ci-dessus, contenant un peptide bi-silylé hybride illustré ci-après, a été préparé :

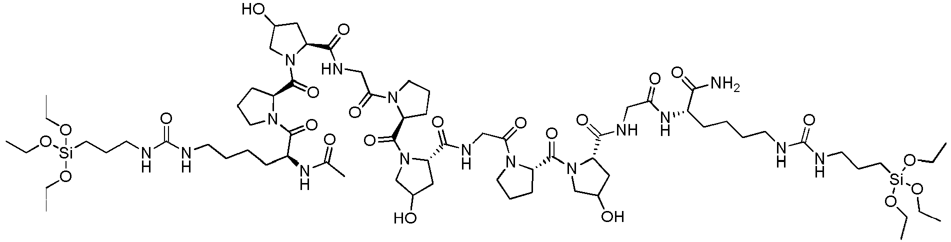

Il a pu être démontré que cet hydrogel possède une structure interne alvéolaire qui peut être propice à la prolifération cellulaire. Des analyses de cryo-microscopie à balayage (MEB) montrent un système alvéolaire multimicrométrique.

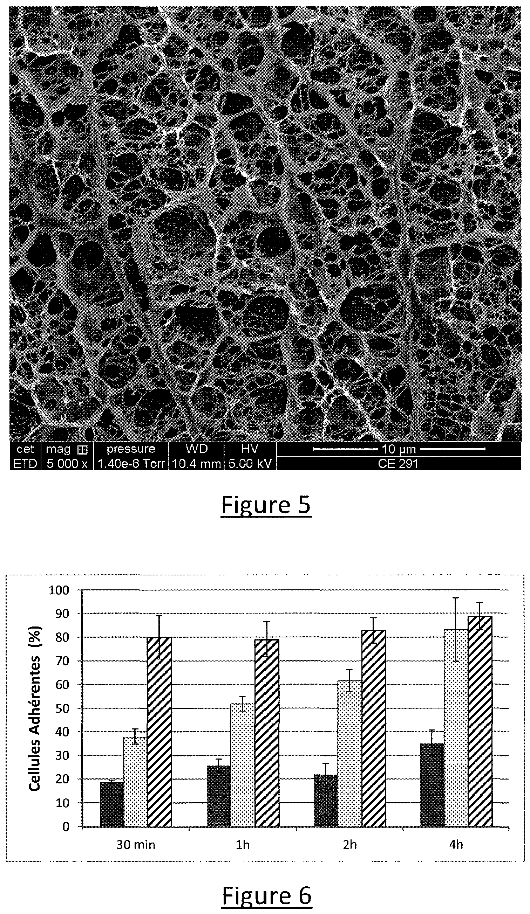

Cela est en effet bien visible sur la Figure 5 illustrant une vue Cryo-MEB de l'hydrogel contenant le peptide bi-silylé préparé.

### Adhésion des cellules

Il a aussi été démontré que l'hydrogel obtenu permet l'adhésion des cellules mMSC (cellules souches mésenchymateuses murines) plus efficacement que le polystyrène traité pour la culture de tissus et après 4h aussi efficacement qu'une mousse de collagène commerciale.

50 µL d'une suspension de cellules à 60 000 cellules par mL ont été déposés sur différents matériaux, y compris l'hydrogel selon l'invention tel que décrit dans cette partie. Le polystyrène traité pour la culture cellulaire (TC-PS) et une mousse commerciale de collagène bovin de type I purifié et réticulé ont été utilisés comme contrôles.

Différents temps d'adhésion à 37°C ont été étudiés avant de retirer le milieu, de rincer avec du DPBS puis de compter les cellules au moyen d'un test de viabilité Cell Titer Glo. Les résultats sont reportés dans la Figure 6.

Cette Figure 6 illustre l'adhésion des cellules mMSC sur l'hydrogel selon l'invention, sur des mousses de collagène et du TC-PS.

### Prolifération cellulaire

L'hydrogel est un aussi bon support que les mousses de collagène pour la prolifération cellulaire.

1000 cellules mMSC ont été déposées sur différents matériaux, y compris l'hydrogel selon l'invention. Le polystyrène traité pour la culture cellulaire (TC-PS) et une mousse commerciale de collagène bovin de type I purifié et réticulé ont été utilisés comme contrôles.

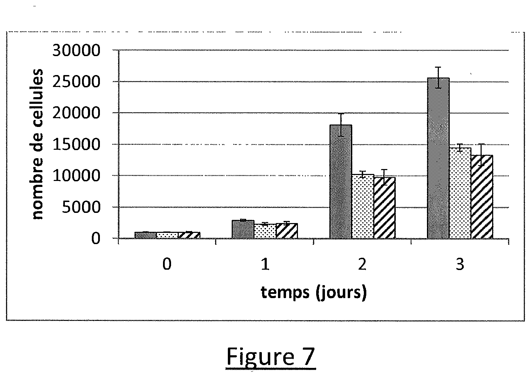

Chaque jour pendant 3 jours, le milieu de culture d'une série d'échantillons est remplacé et les cellules sont comptées au moyen d'un test de viabilité Cell Titer Glo. Les résultats sont reportés dans la Figure 7.

La Figure 7 illustre en effet la prolifération de mMSC (cellules souches mésenchymateuses murines) sur l'hydrogel selon l'invention, sur mousse de collagène et TC-PS.

### Survie des cellules

L'hydrogel permet une bonne survie de cellules emprisonnées à l'intérieur pendant au moins 25 heures

Un avantage majeur du procédé décrit dans le présent brevet est la possibilité d'ajouter des cellules dans le mélange, encore liquide des précurseurs silylés. Ainsi, le gel se forme sans ajout de réactif chimique supplémentaire, en emprisonnant les cellules. Des cellules souches mésenchymateuses murines ont ainsi été encapsulées pendant 25 heures avec une excellente viabilité, comparable au contrôle positif de cellules cultivées en 2D sur une surface de TC-PS.

Le protocole d'encapsulation est le suivant. Une solution d'hydrogel hybride est préparée par solubilisation de 30 mg du peptide hybride de l'exemple 3 dont la structure est rappelée ci-avant, dans 250 µL de milieu de culture DMEM contenant 4,5 g/L de glucose et 0,12 mg/mL de NaF. La solution est incubée à 37°C pendant 17h15. A ce moment-là, la solution est toujours liquide mais sa viscosité a augmenté. 50 µL d'une suspension de cellules mMSC à 500 000 cellules par mL dans du milieu DMEM est ajoutée. La concentration en peptide hybride bi-silylé est alors de 10% massiques. La solution hybride est homogénéisée et 30 µL de cette solution sont déposés dans les puits d'une plaque de culture cellulaire 96 puits. Le gel se forme progressivement à 37°C. 25 heures plus tard, une solution de Calcein-AM et d'Ethidium homodimère III dans le DPBS est ajoutée sur les gels et ces derniers sont analysés par microscopie confocale.

On constate que la majorité des cellules emprisonnées dans l'hydrogel selon l'invention ont été marquées par la calcéine et sont donc vivantes. La viabilité des cellules emprisonnées dans le gel est comparable à la viabilité des cellules déposées sur TC-PS.

### • Conclusion

Il a été démontré la facilité de synthèse et d'utilisation des hydrogels selon la présente invention. Ces hydrogels ont été valablement utilisés avec des molécules de structures chimiques diverses prouvant la flexibilité du procédé selon la présente invention. Il a ainsi pu être préparé des hydrogels présentant des propriétés rhéologiques, biologiques et/ou physico-chimiques satisfaisantes avec très peu de variation (voire aucune variation) des conditions opératoires, hormis la nature des molécules greffées.

## Revendications

1. Procédé de fabrication d'un hydrogel comprenant les étapes de :
- a) polymérisation sol-gel d'au moins une molécule de formule (I) : 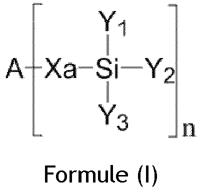 dans laquelle :
• n est un nombre entier supérieur ou égal à 2 ;
• A est un polymère organique choisi parmi les protéines, les peptides tels que des dérivés du collagène, en particulier les séquences comprenant des répétitions de tripeptides Pro-Hyp-Gly ou Pro-Pro-Gly ou Asp-Pro-Gly ou Pro-Lys-Gly, des séquences peptidiques d'autoassemblage comme Arg-Ala-Asp-Ala (SEQ ID 4), des oligoprolines, des oligoalanines, les polysaccharides, tels que l'acide hyaluronique et ses dérivés, les oligonucléotides, des polymères de C₁-C₆-alkylènes-glycols, ou de polyvinylpyrrolidone;
• Xa est une liaison chimique ou un groupement espaceur représenté par un radical divalent dérivé d'une chaîne hydrocarbonée aliphatique saturée ou insaturée comportant de 1 à 10 atomes de carbone, dans laquelle sont éventuellement intercalés, un ou plusieurs chaînons structuraux choisis parmi le groupe arylène, ou les fragments -O-, -S-, - C(=O)-, SO₂ ou -N(R₁)-, ladite chaîne étant non substituée ou substituée par un ou plusieurs radicaux choisis parmi les atomes d'halogène, les groupements hydroxyle, C₁-C₄ alkyle, benzyle et/ou phénéthyle ;
• R₁ représente un atome d'hydrogène, un groupement hydrocarboné aliphatique comportant de 1 à 6 atomes de carbones, un benzyle ou un phénéthyle ;
• Y₁, Y₂, Y₃, identiques ou différents, représentent chacun, indépendamment l'un de l'autre un atome d'hydrogène, d'halogène, un groupement -OR₂, un aryle ou une chaîne hydrocarbonée aliphatique saturée ou insaturée comportant de 1 à 6 atomes de carbone éventuellement substitués par un atome halogène, un groupement aryle ou hydroxyle ;
• R₂ représente un atome d'hydrogène, un groupement aryle ou une chaîne hydrocarbonée aliphatique saturée ou insaturée comportant de 1 à 6 atomes de carbone ;
• au moins deux groupement Xa tels que définis ci-dessus étant reliés à des points d'accroches différents sur A;
- b) mise en présence d'eau, optionnellement conjointement à l'étape a) ; et
- c) récupération de l'hydrogel,
**caractérisé en ce que** le procédé de polymérisation sol-gel est effectué à pH physiologique.

2. Procédé de fabrication d'un hydrogel selon la revendication 1, **caractérisé en ce que** ledit procédé comprend l'ajout conjointement ou successivement à l'étape a) d'au moins un type de molécule de formule (II) : 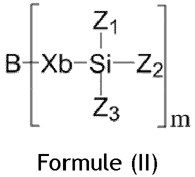 dans laquelle :
• m est un nombre entier supérieur ou égal à 1, préférentiellement égal à 1 ;
• B est un principe actif choisi parmi un peptide, un oligopeptide, une protéine, telle que le collagène, un acide désoxyribonucléique, un acide ribonucléique, un polysaccharide, tel qu'une pectine, un chitosane, un acide hyaluronique, un polysaccharide polyarabinose et polygalactose, et un glycolipide;
• Xb est une liaison chimique ou un groupement espaceur représenté par un radical divalent dérivé d'une chaîne hydrocarbonée aliphatique saturée ou insaturée comportant de 1 à 10 atomes de carbone, dans laquelle sont éventuellement intercalés, un ou plusieurs chaînons structuraux choisis parmi le groupe arylène, ou les fragments -O-, -S-, - C(=O)-, SO₂ ou -N(R₃)-, ladite chaîne étant non substituée ou substituée par un ou plusieurs radicaux choisis parmi les atomes d'halogène, les groupements hydroxyle, C₁-C₄ alkyle, benzyle et/ou phénéthyle ;
• R₃ représente un atome d'hydrogène, un groupement hydrocarboné aliphatique comportant de 1 à 6 atomes de carbones, un benzyle ou un phénéthyle ;
• Z₁, Z₂, Z₃, identiques ou différents, représentent chacun, indépendamment l'un de l'autre un atome d'hydrogène, d'halogène, un groupement -OR₄, un aryle ou une chaîne hydrocarbonée aliphatique saturée ou insaturée comportant de 1 à 6 atomes de carbone éventuellement substitués par un atome halogène, un groupement aryle ou hydroxyle ;
• R₄ représente un atome d'hydrogène, un groupement aryle ou une chaîne hydrocarbonée aliphatique saturée ou insaturée comportant de 1 à 6 atomes de carbone ; et
• avec préférentiellement un seul des groupements Z₁, Z₂, ou Z₃ comme atome d'halogène ou groupement OR₄.

3. Procédé de fabrication d'un hydrogel selon l'une quelconque des revendications 1 ou 2 **caractérisé en ce que** ledit hydrogel est polymérisé sur ou dans au moins un premier hydrogel en tant que support, résultant ainsi en un hydrogel multicouches.

4. Hydrogel obtenu par le procédé selon l'une quelconque des revendications 1 à 3.

5. Hydrogel selon la revendication 4 pour son utilisation à des fins thérapeutiques et/ou chirurgicales, préférentiellement **caractérisé en ce que** ledit hydrogel permet la délivrance et/ou le transport de molécules actives, ou pour son utilisation *in vivo* en ingénierie tissulaire.

6. Utilisation *in vitro* d'un hydrogel selon la revendication 4, en ingénierie tissulaire.

## Patentansprüche

1. Verfahren zur Herstellung eines Hydrogels, umfassend die folgenden Schritte:
- a) Sol-Gel-Polymerisation von mindestens einem Molekül der Formel (I): 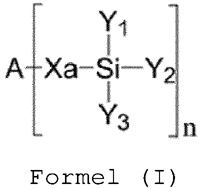 wobei:
• n eine ganze Zahl größer oder gleich 2 ist;
• A ein organisches Polymer ist, ausgewählt aus Proteinen, Peptiden, wie beispielsweise Kollagenderivaten, insbesondere Sequenzen umfassend Wiederholungen von Tripeptiden Pro-Hyp-Gly oder Pro-Pro-Gly oder Asp-Pro-Gly oder Pro-Lys-Gly, selbstassemblierenden Peptidsequenzen wie Arg-Ala-Asp-Ala (SEQ ID 4), Oligoprolinen, Oligoalaninen, Polysacchariden, wie Hyaluronsäure und ihre Derivate, Oligonukleotiden, Polymeren von C₁-C₆-Alkylenglykolen oder Polyvinylpyrrolidonen;
• Xa eine chemische Bindung oder eine Spacergruppe ist, die durch einen zweiwertigen Rest repräsentiert wird, der von einer gesättigten oder ungesättigten aliphatischen Kohlenwasserstoffkette mit 1 bis 10 Kohlenstoffatomen abgeleitet ist, in die gegebenenfalls ein oder mehrere Strukturelemente, ausgewählt aus der Arylengruppe oder den Fragmenten -O-, -S-, -C(=O)-, SO₂ oder -N(R₁)-, eingefügt sind, wobei die Kette unsubstituiert oder durch einen oder mehrere Reste, ausgewählt aus Halogenatomen, Hydroxy-, C₁-C₄-Alkyl-, Benzyl- und/oder Phenethylgruppen, substituiert ist;
• R₁ ein Wasserstoffatom oder eine aliphatische Kohlenwasserstoffgruppe mit 1 bis 6 Kohlenstoffatomen, einem Benzyl oder einem Phenethyl repräsentiert;
• Y₁, Y₂, Y₃, die gleich oder verschieden sind, jeweils unabhängig voneinander ein Wasserstoffatom, ein Halogenatom, eine -OR₂-Gruppe, ein Aryl oder eine gesättigte oder ungesättigte aliphatische Kohlenwasserstoffkette mit 1 bis 6 Kohlenstoffatomen repräsentieren, die gegebenenfalls durch ein Halogenatom, eine Aryl- oder Hydroxygruppe substituiert ist;
• R₂ ein Wasserstoffatom, eine Arylgruppe oder eine gesättigte oder ungesättigte aliphatische Kohlenwasserstoffkette mit 1 bis 6 Kohlenstoffatomen repräsentiert;
• mindestens zwei Gruppen Xa wie oben definiert, die an verschiedenen Befestigungspunkten mit A verbunden sind;
- b) Inkontaktbringen mit Wasser, gegebenenfalls in Verbindung mit Schritt a); und
- c) Rückgewinnung des Hydrogels,
**dadurch gekennzeichnet, dass** das Verfahren der Sol-Gel-Polymerisation bei physiologischem pH-Wert durchgeführt wird.

2. Verfahren zur Herstellung eines Hydrogels nach Anspruch 1, **dadurch gekennzeichnet, dass** das Verfahren die gleichzeitige oder aufeinanderfolgende Zugabe in Schritt a) mindestens einer Art von Molekülen der Formel (II) umfasst: 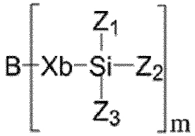 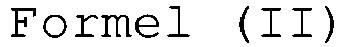 wobei:
• m eine ganze Zahl größer oder gleich 1, vorzugsweise gleich 1, ist;
• B ein Wirkstoff ist, ausgewählt aus einem Peptid, einem Oligopeptid, einem Protein wie Kollagen, einer Desoxyribonukleinsäure, einer Ribonukleinsäure, einem Polysaccharid wie Pektin, Chitosan, Hyaluronsäure, einem Polyarabinose- und Polygalactose-Polysaccharid und einem Glykolipid;
• Xb eine chemische Bindung oder eine Spacergruppe ist, die durch einen zweiwertigen Rest repräsentiert wird, der aus einer gesättigten oder ungesättigten aliphatischen Kohlenwasserstoffkette mit 1 bis 10 Kohlenstoffatomen abgeleitet wird, in die gegebenenfalls ein oder mehrere Strukturelemente, ausgewählt aus der Arylengruppe oder den Fragmenten -O-, -S-, -C(=O)-, SO₂ oder -N(R₃)-, eingefügt sind, wobei die Kette unsubstituiert oder durch einen oder mehrere Reste, ausgewählt aus Halogenatomen, Hydroxy-, C₁-C₄-Alkyl-, Benzyl- und/oder Phenethylgruppen substituiert ist;
• R₃ ein Wasserstoffatom oder eine aliphatische Kohlenwasserstoffgruppe mit 1 bis 6 Kohlenstoffatomen, einem Benzyl oder einem Phenethyl repräsentiert;
• Z₁, Z₂, Z₃, die gleich oder verschieden sind, jeweils unabhängig voneinander ein Wasserstoffatom, ein Halogenatom, eine -OR₄-Gruppe, ein Aryl oder eine gesättigte oder ungesättigte aliphatische Kohlenwasserstoffkette mit 1 bis 6 Kohlenstoffatomen repräsentieren, die gegebenenfalls durch ein Halogenatom, eine Aryl- oder Hydroxygruppe substituiert ist;
• R₄ ein Wasserstoffatom, eine Arylgruppe oder eine gesättigte oder ungesättigte aliphatische Kohlenwasserstoffkette mit 1 bis 6 Kohlenstoffatomen repräsentiert; und
• mit vorzugsweise einer einzigen der Gruppen Z₁, Z₂, oder Z₃ wie Halogenatom oder OR₄-Gruppe.

3. Verfahren zur Herstellung eines Hydrogels nach einem der Ansprüche 1 oder 2, **dadurch gekennzeichnet, dass** das Hydrogel auf oder in mindestens einem ersten Hydrogel als Träger polymerisiert wird, so dass sich ein mehrschichtiges Hydrogel ergibt.

4. Hydrogel, das mit dem Verfahren nach einem der Ansprüche 1 bis 3 erhalten wird.

5. Hydrogel nach Anspruch 4 zur Verwendung für therapeutische und/oder chirurgische Zwecken, vorzugsweise **dadurch gekennzeichnet, dass** das Hydrogel die Abgabe und/oder den Transport von aktiven Molekülen oder zur *In-vivo-*Verwendung beim Tissue Engineering ermöglicht.

6. *In*-*vitro*-Verwendung eines Hydrogels nach Anspruch 4 beim Tissue Engineering.

## Claims

1. A process for producing a hydrogel comprising the steps of:
- a) sol-gel polymerization of at least one molecule of formula (I): 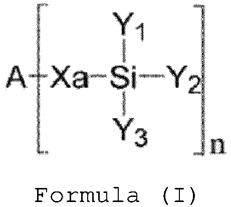 wherein:
• n is an integer greater than or equal to 2;
• A is an organic polymer selected from proteins, peptides such as collagen derivatives, in particular sequences comprising Pro-Hyp-Gly or Pro-Pro-Gly or Asp-Pro-Gly or Pro-Lys-Gly tripeptide repeats, self-assembling peptide sequences such as Arg-Ala-Asp-Ala (SEQ ID 4), oligoprolines, oligoalanines, polysaccharides such as hyaluronic acid and derivatives thereof, oligonucleotides, C₁-C₆-alkylene-glycol polymers, or polyvinylpyrrolidone;
• Xa is a chemical bond or a spacer group represented by a divalent radical derived from a saturated or unsaturated aliphatic hydrocarbon chain comprising from 1 to 10 carbon atoms, optionally intercalated with one or more structural linkers selected from arylene or fragments -O-, -S-, -C(=O)-, SO₂ or -N(R₁)-, wherein said chain is unsubstituted or is substituted by one or more radicals selected from halogen atoms, a hydroxyl group, a C₁-C₄ alkyl group, a benzyl group and/or a phenethyl group;
• R₁ represents a hydrogen atom, an aliphatic hydrocarbon group comprising from 1 to 6 carbon atoms, a benzyl or a phenethyl;
• Y₁, Y₂, Y₃, which may be identical or different, each independently represent a hydrogen atom, a halogen atom, an -OR₂ group, an aryl or a saturated or unsaturated aliphatic hydrocarbon chain comprising from 1 to 6 carbon atoms optionally substituted by a halogen atom, an aryl group or a hydroxyl group;
• R₂ represents a hydrogen atom, an aryl group or a saturated or unsaturated aliphatic hydrocarbon chain comprising from 1 to 6 carbon atoms;
• wherein at least two Xa groups as defined above are linked to different attachment points on A;
- b) mixing with water, optionally at the same time as step a); and
- c) recovering the hydrogel,
**characterized in that** the sol-gel polymerization process is carried out at physiological pH.

2. The process for producing a hydrogel as claimed in claim 1, **characterized in that** said process comprises the addition, at the same time as or subsequent to step a), of at least one type of molecule of formula (II): 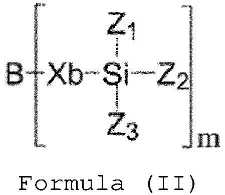 wherein:
• m is an integer greater than or equal to 1, preferentially equal to 1;
• B is an active ingredient selected from a peptide, an oligopeptide, a protein, such as collagen, a deoxyribonucleic acid, a ribonucleic acid, a polysaccharide, such as a pectin, a chitosan, a hyaluronic acid, a polyarabinose and polygalactose polysaccharide, and a glycolipid;
• Xb is a chemical bond or a spacer group represented by a divalent radical derived from a saturated or unsaturated aliphatic hydrocarbon chain comprising from 1 to 10 carbon atoms, optionally intercalated with one or more structural linkers selected from arylene or fragments -O-, -S-, -C(=O)-, SO₂ or -N(R₃)-, wherein said chain is unsubstituted or is substituted by one or more radicals selected from halogen atoms, a hydroxyl group, a C₁-C₄ alkyl group, a benzyl group and/or a phenethyl group;
• R₃ represents a hydrogen atom, an aliphatic hydrocarbon group comprising from 1 to 6 carbon atoms, a benzyl or a phenethyl;
• Z₁, Z₂, Z₃, which may be identical or different, each independently represent a hydrogen atom, a halogen atom, an -OR₄ group, an aryl or a saturated or unsaturated aliphatic hydrocarbon chain comprising from 1 to 6 carbon atoms optionally substituted by a halogen atom, an aryl group or a hydroxyl group;
• R₄ represents a hydrogen atom, an aryl group or a saturated or unsaturated aliphatic hydrocarbon chain comprising from 1 to 6 carbon atoms; and
• wherein preferentially only one of the Z₁, Z₂, or Z₃ groups is a halogen atom or an OR₄ group.

3. The process for producing a hydrogel as claimed in any one of claims 1 or 2, **characterized in that** said hydrogel is polymerized on or in at least a first hydrogel as a support, thus resulting in a multilayer hydrogel.

4. A hydrogel obtained by the process as claimed in any one of claims 1 to 3.

5. The hydrogel as claimed in claim 4 for therapeutic and/or surgical use, preferentially **characterized in that** said hydrogel allows the delivery and/or transport of active molecules, or for use *in vivo* in tissue engineering.

6. *In vitro* use of a hydrogel as claimed in claim 4, in tissue engineering.
